(19) Europäisches Patentamt
European Patent Office
Office européen des brevets

(11) EP 4 509 043 A1

(12) EUROPEAN PATENT APPLICATION
published in accordance with Art. 153(4) EPC

(43) Date of publication:
19.02.2025 Bulletin 2025/08

(21) Application number: 23889996.7

(22) Date of filing: 13.02.2023

(51) International Patent Classification (IPC):
A61B 5/024 (2006.01)      A61B 5/0245 (2006.01)
A61B 5/0205 (2006.01)     A61B 5/346 (2021.01)
A61B 5/349 (2021.01)

(52) Cooperative Patent Classification (CPC):
A61B 5/00; A61B 5/0205; A61B 5/024;
A61B 5/0245; A61B 5/11; A61B 5/16; A61B 5/318;
A61B 5/346; A61B 5/349; A61B 5/389

(86) International application number:
PCT/CN2023/075666

(87) International publication number:
WO 2024/103547 (23.05.2024 Gazette 2024/21)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 17.11.2022 PCT/CN2022/132671

(71) Applicant: Shenzhen Shokz Co., Ltd.
Shenzhen, Guangdong 518108 (CN)

(72) Inventors:
• ZHOU, Xin
Shenzhen, Guangdong 518108 (CN)

• LIU, Jia
Shenzhen, Guangdong 518108 (CN)
• LI, Meiqi
Shenzhen, Guangdong 518108 (CN)
• SU, Lei
Shenzhen, Guangdong 518108 (CN)
• ZHANG, Yuxiang
Shenzhen, Guangdong 518108 (CN)
• LIAO, Fengyun
Shenzhen, Guangdong 518108 (CN)
• QI, Xin
Shenzhen, Guangdong 518108 (CN)

(74) Representative: Wang, Bo
Panovision IP
Ebersberger Straße 3
85570 Markt Schwaben (DE)

(54) WEARABLE DEVICE

(57) The present disclosure provides a wearable device. The wearable device comprises at least two electrodes, configured to be attached to a human skin to collect an electrocardiograph (ECG) signal of a human body; a wearable structure, configured to carry the at least two electrodes and attach the at least two electrodes to two sides of a midsagittal plane of the human body; and a processor, configured to determine a heart rate variability (HRV) of the human body based on the ECG signal, and determine a physical state of the human body based at least on the HRV.

200

Electrode
210

Wearable structure
220

Processor
230

FIG. 2

EP 4 509 043 A1

**Description**

**CROSS-REFERENCE TO RELATED APPLICATIONS**

This application claims priority to International Application No. PCT/CN2022/132671, filed on November 17, 2022, the entire contents of which are incorporated herein by reference.

**TECHNICAL FIELD**

The present disclosure relates to the field of wearable devices, and in particular to a wearable device capable of determining a physical state.

**BACKGROUND**

Current wearable devices designed to monitor human physical conditions have extensively researched parameters such as body temperature, heart rate, and electromyography. However, for users who engage in static activities like yoga, Pilates, meditation, seated meditation, and breathing exercises, maintaining a relaxed physical state is essential to achieve desired body and mind conditioning effects. Unfortunately, existing wearable devices are unable to accurately assess the user's physical state during these activities, nor can they provide guidance for adjusting the physical state as needed.

Consequently, there is a need for a wearable device that can both determine and assist in adjusting the user's physical state effectively.

**SUMMARY**

One of the embodiments of the present disclosure provides a wearable device. The wearable device may comprise at least two electrodes, configured to be attached to a human skin to collect an electrocardiograph (ECG) signal of a human body; a wearable structure, configured to carry the at least two electrodes and attach the at least two electrodes to two sides of a midsagittal plane of the human body; and a processor, configured to determine a heart rate variability (HRV) of the human body based on the ECG signal, and determine a physical state of the human body based at least on the HRV.

**BRIEF DESCRIPTION OF THE DRAWINGS**

The present disclosure will be further illustrated by way of exemplary embodiments, which will be described in detail by means of the accompanying drawings. These embodiments are not limiting, and in these embodiments, the same numbering indicates the same structure, wherein:

FIG. 1 is a schematic diagram illustrating an exemplary application scenario of a wearable device according to some embodiments of the present disclosure;
FIG. 2 is a schematic structural diagram illustrating an exemplary wearable device according to some embodiments of the present disclosure;
FIG. 3 is a schematic plane diagram illustrating an exemplary wearable device according to some embodiments of the present disclosure;
FIG. 4 is a schematic structural diagram illustrating an exemplary wearable device according to some embodiments of the present disclosure;
FIG. 5 is a schematic structural diagram illustrating an exemplary wearable device according to some embodiments of the present disclosure;
FIG. 6 is a schematic diagram illustrating an exemplary human body;
FIG. 7 is a schematic diagram illustrating an exemplary ilium of a waist region of a human body;
FIG. 8 is a schematic structural diagram illustrating an exemplary wearable device according to some embodiments of the present disclosure;
FIG. 9 is a schematic structural diagram illustrating a front side of trousers according to some embodiments of the present disclosure;
FIG. 10 is a schematic structural diagram illustrating a back side of trousers according to some embodiments of the present disclosure;
FIG. 11 is a schematic diagram illustrating a standard electrocardiogram according to some embodiments of the present disclosure;

FIG. 12 is a schematic diagram illustrating an exemplary process of determining a physical state of a human body based on a heart rate variability (HRV) according to some embodiments of the present disclosure;

FIG. 13 is a schematic diagram illustrating an exemplary process of determining a physical state of a human body based on an HRV and a heart rate according to some embodiments of the present disclosure;

FIG. 14 is a schematic diagram illustrating an exemplary process of determining a physical state of a human body based on an HRV and respiratory state information according to some embodiments of the present disclosure; and

FIG. 15 is a schematic structural diagram illustrating another exemplary wearable device according to some embodiments of the present disclosure.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

The embodiments of the present disclosure provide a wearable device. The wearable device may comprise: at least two electrodes, configured to be attached to a human skin to collect an electrocardiograph (ECG) signal of a human body; a wearable structure, configured to carry the at least two electrodes and attach the at least two electrodes to two sides of a midsagittal plane of the human body; and a processor, configured to determine a heart rate variability (HRV) of the human body based on the ECG signal, and determine a physical state of the human body based at least on the HRV. The wearable device provided by the present disclosure determines the physical state of the human body by real-time detection of parameters such as the HRV, the heart rate, and respiratory state information of the human body, and provide real-time feedback of the physical state and reference health advice to the user, thereby realizing a closed loop of a health monitoring process for the user. In addition, the physical state of the user includes a tense state, a relaxed state, etc., and the determined physical state is applied to a plurality of fields such as physical exercise, psychological testing, clinical health monitoring, etc.

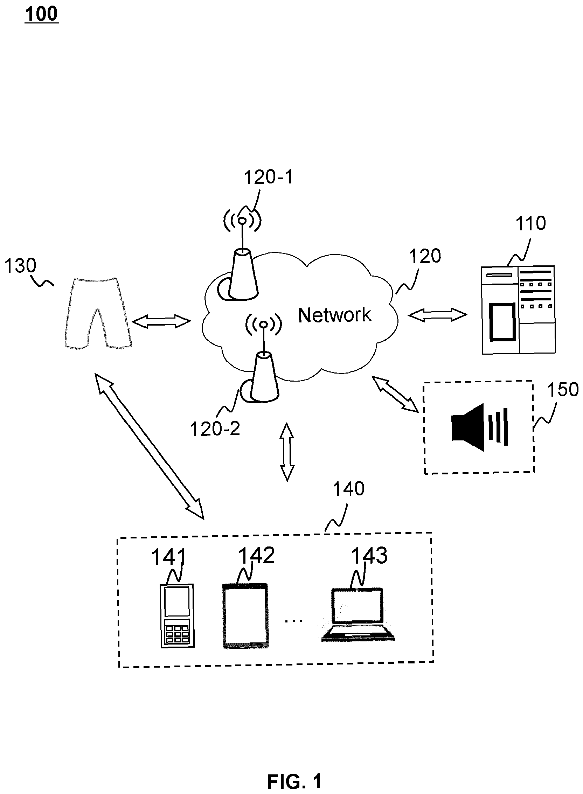

FIG. 1 is a schematic diagram illustrating an exemplary application scenario of a wearable device according to some embodiments of the present disclosure.

As shown in FIG. 1, an application scenario 100 may include a processing device 110, a network 120, a wearable device 130, a terminal device 140, and a feedback module 150. In some embodiments, the application scenario 100 may be applied to various scenarios such as physical exercise, psychological testing, clinical health monitoring, etc.

In some embodiments, the application scenario 100 may be configured to measure a physical state of a user during exercise of the user. Exemplary exercises may include yoga, Pilates, meditation, sitting meditation, standing meditation, breathing training, etc. Exemplary physical states may include a relaxed state and a tense state.

In some embodiments, the processing device 110 may include a processor. In some embodiments, the processing device 110 may be configured to process electrical signals generated by the wearable device 130 and the terminal device 140. For example, the processing device 110 may receive an electrocardiograph (ECG) signal transmitted by the wearable device 130 via the network 120. The processing device 110 may receive input information input by the user through the terminal device 140 via the network 120. In some embodiments, the processing device 110 may determine a heart rate variability (HRV) of a human body based on the ECG signal, and determine the physical state of the human body at least based on the HRV. In some embodiments, the processing device 110 may determine the HRV and a heart rate of the human body based on the ECG signal, and determine the physical state of the human body at least based on the HRV and the heart rate. In some embodiments, the processing device 110 may use at least the HRV as input data into a trained machine learning model, and the trained machine learning model may output the physical state of the human body based on the input data. In some embodiments, the processing device 110 may determine a calibration curve, and determine the physical state of the human body based on the calibration curve. In some embodiments, the processing device 110 may determine the physical state of the human body based on the HRV and respiratory state information. In some embodiments, the processing device 110 may use at least the HRV and the respiratory state information as input data into the trained machine learning model, and the trained machine learning model may output the physical state of the human body based on the input data. In some embodiments, the processing device 110 may recognize an exercise movement of a corresponding part of the human body based on a second strain sensor. The exercise movement of the corresponding part may be related to a position of the second strain sensor. For example, when the second strain sensor is disposed at a position corresponding to a leg or a buttock of the human body, the processing device 110 may recognize the exercise movement of lower limbs of the human body based on the second strain sensor. In some embodiments, the processing device 110 may issue a control instruction in response to the physical state of the human body.

In some embodiments, the processing device 110 may be local or remote. For example, the processing device 110 may access information stored in the wearable device 130 and/or the terminal device 140 directly or via the network 120. In some embodiments, the processing device 110 may be directly connected with the wearable device 130 and/or the terminal device 140 to access the information stored therein. For example, the processing device 110 may be disposed in the wearable device 130 and implement information interaction with the terminal device 140 via the network 120. As another example, the processing device 110 may be disposed in the terminal device 140 and implement information interaction with the wearable device 130 via the network 120. In some embodiments, the processing device 110 may be implemented on a cloud platform. For example, the cloud platform may include any one of a private cloud, a public cloud, a hybrid cloud, a community cloud, a decentralized cloud, an internal cloud, or the like, or any combination thereof.

In some embodiments, the processing device 110 may include one or more sub-processing devices (e.g., a single-core processing device or a multi-core processing device). Merely by way of example, the processing device 110 may include a central processing unit (CPU), an application-specific integrated circuit (ASIC), an application-specific instruction processor (ASIP), a graphics processing unit (GPU), a physical processing unit (PPU), a digital signal processor (DSP), a field programmable gate array (FPGA), a programmable logic device (PLD), a controller, a microcontroller unit, a reduced instruction set computer (RISC), a microprocessor, or the like, or any combination thereof.

The network 120 can facilitate the exchange of data and/or information between components in the application scenario 100. In some embodiments, one or more components (e.g., the processing device 110, the wearable device 130, the terminal device 140, and the feedback module 150) in the application scenario 100 may send data and/or information to other components in the application scenario 100 via the network 120. For example, the electrical signal generated by the terminal device 140 may be transmitted to the processing device 110 via the network 120. In some embodiments, the network 120 may be any type of wired or wireless network. For example, the network 120 may include a cable network, a wired network, a fiber optic network, a telecommunications network, an internal network, an internet network, a local area network (LAN), a wide area network (WAN), a wireless area network (WLAN), a metropolitan area network (MAN), a public switched telephone network (PSTN), a Bluetooth network, a ZigBee network, a near field communication (NFC) network, or the like, or any combination thereof. In some embodiments, the network 120 may include one or more network access points. For example, the network 120 may include wired or wireless network access points, such as base stations and/or Internet exchange points 120-1, 120-2, ..., via which the one or more components in the application scenario 100 may be connected to the network 120 to exchange data and/or information.

The wearable device 130 refers to clothing or equipment with a wearable function. In some embodiments, the wearable device 130 may include but is not limited to trousers, a belt, etc. The wearable device 130 may be configured to obtain an ECG signal and an electromyographic signal of the user, and transmit the ECG signal and the electromyographic signal to the processing device 110 via the network 120. In some embodiments, the wearable device 130 may be provided with at least two electrodes. The at least two electrodes may be configured to be attached to a human skin to collect the ECG signal of the human body. In some embodiments, the wearable device 130 may include a first strain sensor. The first strain sensor may be configured to collect the respiratory state information of the human body based on a fluctuation of a waist region when the human body breathes. In some embodiments, the wearable device 130 may include a second strain sensor. The second strain sensor may be disposed at a leg, a buttock, an arm, a shoulder, a hand, or the like, of the user. Merely by way of example, the second strain sensor may be disposed at the leg or the buttock of the user, and the second strain sensor may be configured to obtain a lower limb movement parameter of the human body. Exemplary lower limb movement parameters may include a joint bending angle, a bending direction, etc. As another example, the second strain sensor may be disposed at the arm or the shoulder of the user, and the second strain sensor may be configured to obtain an arm movement parameter (e.g., an arm bending angle, a bending direction, etc.). In some embodiments, the wearable device 130 may include an electromyographic module. The electromyographic module may be configured to collect the electromyographic signals of the human body.

It should be noted that the wearable device 130 is not limited to the trousers shown in FIG. 1, but may also include other devices, such as the belt, a skirt, a wrist guard, an elbow pad, a shoulder pad, a knee pad, a sock, etc., which are not limited here. Any device that can use the present disclosure is within the scope of protection of the present disclosure.

The terminal device 140 refers to a device that interacts with the user. In some embodiments, the terminal device 140 may be integrated into the wearable device 130. The terminal device 140 may receive input information input by the user and send the input information to the processing device 110 via the network 120. In some embodiments, the terminal device 140 may receive feedback information sent by the processing device 110 via the network 120 and present the feedback information to the user. Exemplary feedback information may include information about a current physical state, an exercise suggestion, a health reminder, or the like. In some embodiments, the terminal device 140 may be a mobile smart terminal 141, a tablet computer 142, a laptop computer 143, or the like. In some embodiments, the terminal device 140 may be integrated into the processing device 110 and the wearable device 130. In some embodiments, the terminal device 140 may be other devices, such as a mobile phone, a smart home device, a smart mobile device, a virtual reality (VR) device, an augmented reality (AR) device, or the like, or any combination thereof. In some embodiments, the smart home device may include a control device for a smart appliance, a smart monitoring device, a smart TV, a smart camera, or the like, or any combination thereof. In some embodiments, the smart mobile device may include a smart phone, a personal digital assistant (PDA), a gaming device, a navigation device, a POS device, or the like, or any combination thereof. In some embodiments, the VR device and/or the AR device may include a VR helmet, VR glasses, VR goggles, an AR helmet, AR glasses, AR goggles, or the like, or any combination thereof.

The feedback module 150 may be configured to send the feedback information to the user based on the control instruction. In some embodiments, the feedback module 150 may be a loudspeaker, an electronic screen, a vibration sensing device, etc. In some embodiments, the feedback module 150 may be a part of the terminal device 140, or integrated into the terminal device 140. In some embodiments, the feedback module 150 may also be integrated into the wearable device 130 and is arranged independently relative to the terminal device 140.

In some embodiments, the application scenario 100 may include other devices or components, such as a database.

The database may be configured to store data, such as physiological parameter information (e.g., the ECG signal, the electromyographic signal, the heart rate, the HRV, the respiratory status information, etc.) received by the terminal device 140. In some embodiments, the database may store information obtained from the wearable device 130 and/or the mobile terminal device. In some embodiments, the database may include a mass memory, a removable memory, a volatile read-write memory (e.g., a random access memory (RAM)), a read-only memory (ROM), or the like, or any combination thereof. In some embodiments, the database may be connected to the network 120 to communicate with the one or more components (e.g., the processing device 110, the wearable device 130, the terminal device 140, the mobile terminal device, etc.) in the application scenario 100. The one or more components in the application scenario 100 may access the data stored in the database via the network 120. In some embodiments, the database may be a part of the processing device 110.

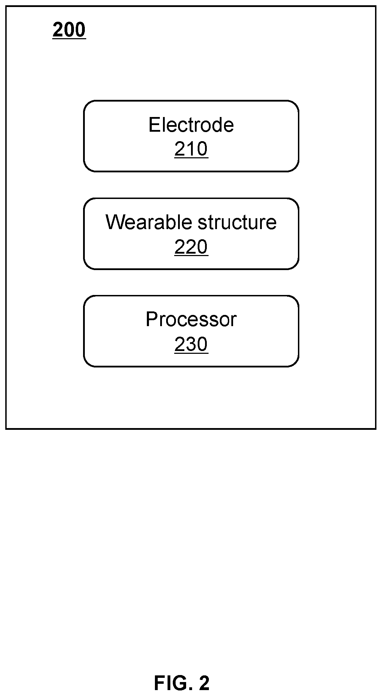

FIG. 2 is a schematic structural diagram illustrating an exemplary wearable device according to some embodiments of the present disclosure.

As shown in FIG. 2, a wearable device 200 may include at least two electrodes 210, a wearable structure 220, and a processor 230. The at least two electrodes 210 may be configured to collect an ECG signal of a human body. The processor 230 may be configured to process the ECG signal into information such as a heart rate and an HRV, and determine a physical state of a human body based on the information.

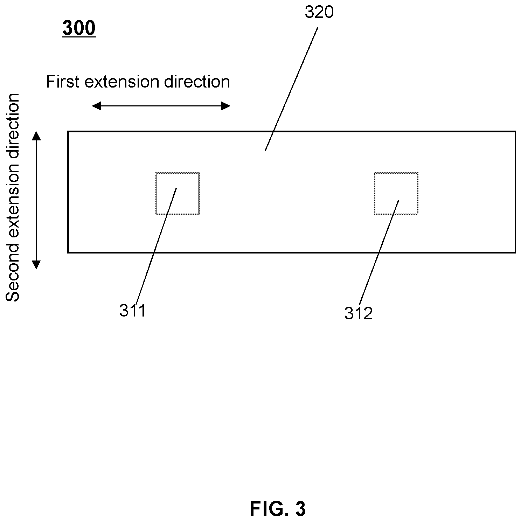

The at least two electrodes 210 may be configured to be attached to a human skin to collect the ECG signal of the human body. In some embodiments, two, three, or other numbers of electrodes 210 may be provided. In some embodiments, the at least two electrodes 210 may be located on two sides of a midsagittal plane of the human body. For example, the at least two electrodes 210 may be attached to an ilium, a back waist, an abdomen, legs, or other regions on two sides of the midsagittal plane of the human body to collect the ECG signal of the human body. In some embodiments, the at least two electrodes 210 may be fixed to the wearable structure 220 by means of a touch fastener, a mesh bag, a buckle, hot pressing, gluing, suturing, etc. In some embodiments, the at least two electrodes 210 may include a first electrode and a second electrode. The first electrode and the second electrode may be located on the two sides of the midsagittal plane of the human body, respectively. More descriptions regarding the at least two electrodes 210 may be found in FIG. 3 and related descriptions thereof.

The wearable structure 220 may be configured to carry the at least two electrodes 210, and attach the at least two electrodes 210 to regions of the two sides of the midsagittal plane of the human body. The wearable structure 220 may be made of comfortable and breathable fabrics, such as cotton, linen, nylon, or the like. In some embodiments, the wearable structure 220 may be one of trousers (e.g., shorts, trousers, and jumpsuits), a belt, a skirt, a knee pad, a waist pad, or any combination thereof. More descriptions regarding the wearable structure 220 may be found in FIGs. 3-5 and related descriptions thereof.

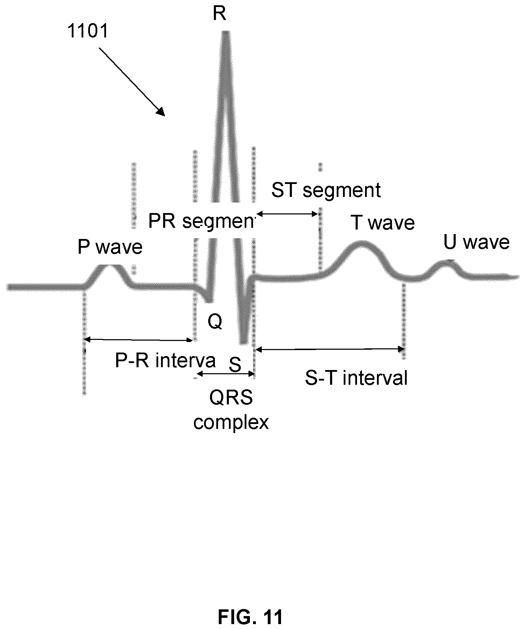

The processor 230 may be configured to determine the HRV of the human body based on the ECG signal, and determine the physical state of the human body at least based on the HRV. The processor 230 may be integrated in any position of the wearable structure 220, or may be independently disposed relative to the wearable structure 220. For example, the processor 230 may be disposed in a cloud server. In some embodiments, the processor 230 may determine the HRV of the human body based on the ECG signal, and determine the physical state of the human body at least based on the HRV. In some embodiments, the processor 230 may determine the HRV and the heart rate of the human body based on the ECG signal, and determine the physical state of the human body based on the HRV and the heart rate. In some embodiments, the processor 230 may use at least the HRV as input data into a trained machine learning model, and the trained machine learning model may output the physical state of the human body based on the input data. In some embodiments, the processor 230 may determine a calibration curve, and determine the physical state of the human body based on the calibration curve. In some embodiments, the processor 230 may determine the physical state of the human body based on the HRV and the respiratory state information. In some embodiments, the processor 230 may use at least the HRV and the respiratory state information as input data into the trained machine learning model, and the trained machine learning model may output the physical state of the human body based on the input data. The machine learning model outputs the physical state of the human body based on the input data. In some embodiments, the processor 230 may recognize an exercise movement of lower limbs of the human body based on a second strain sensor. In some embodiments, the processor 230 may issue a control instruction in response to the physical state of the human body. More descriptions regarding the processor 230 may be found in FIGs. 11-16 and related descriptions thereof.

The HRV can reflect a change in a time interval between heartbeats. A healthy heart has an irregularity, and the irregularity of the heart is controlled by an autonomic nervous system. The HRV can reflect the health of the nervous system. There are two types of autonomic nerves, one is sympathetic nerves that control "fight or escape", and the other is parasympathetic nerves that control "relaxation or digestion". Under the control of the sympathetic nerves, the HRV decreases, while under the control of the parasympathetic nerves, the HRV increases. When the human body is relaxed, the time interval between the heartbeats varies greatly. When the heart is highly nervous, the time interval between the heartbeats is small, and even regular beating with an equal time interval can be achieved.

An analysis method of the HRV may include a linear analysis method and a nonlinear analysis method. The linear analysis method may include time domain analysis and frequency domain analysis. The time domain analysis may further include statistical analysis, geometrical analysis, etc. The nonlinear analysis method may include an image method and a nonlinear parameter calculation method. The image method may include an electrocardiogram scatter plot method. The nonlinear analysis method may include a fractal dimension (correlation dimension, Hausdorff dimension, or information dimension) analysis method, a complexity analysis method, a Lyapunov index, a Kolmogorov entropy, approximate entropy analysis, etc. The study of the HRV can well reflect the changes of some physiological data during the interaction between the wearable device and the human body.

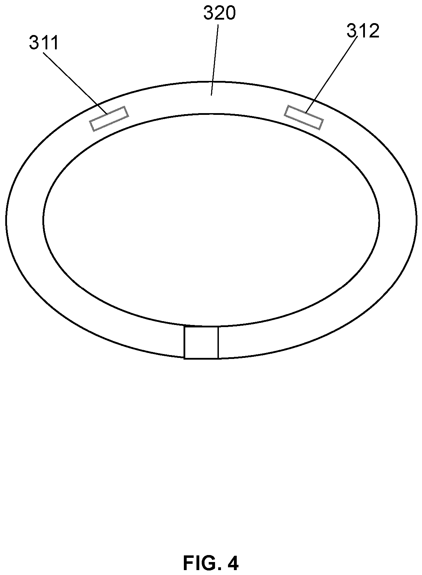

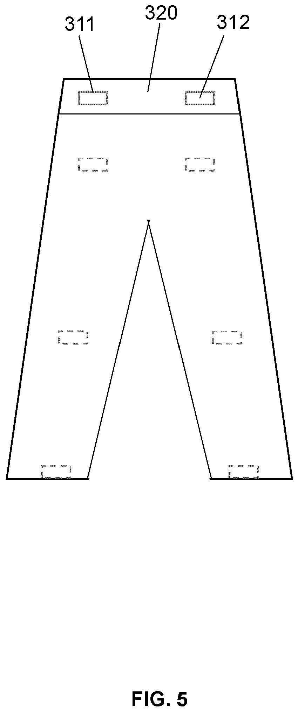

FIG. 3 is a schematic plane diagram illustrating an exemplary wearable device according to some embodiments of the present disclosure. FIG. 4 and FIG. 5 are schematic structural diagram illustrating an exemplary wearable device according to some embodiments of the present disclosure.

As shown in FIGs. 3-5, a wearable device 300 may include a first electrode 311, a second electrode 312, and a wearable structure 320.

The first electrode 311 and the second electrode 312 may be configured to be attached to a human skin to collect an ECG signal of the human body. In some embodiments, the first electrode 311 and the second electrode 312 may be attached to different parts of the human body. A part to which the first electrode 311 is attached may have a first potential, and a part to which the second electrode 312 is attached may have a second potential. A difference between the first potential and the second potential may be configured to reflect the ECG signal of the human body.

The wearable structure 320 may be configured to carry the first electrode 311 and the second electrode 312, and attach the first electrode 311 and the second electrode 312 to two sides of a midsagittal plane of the human body. The first electrode 311 and the second electrode 312 may be distributed on the wearable structure 320 at interval. When the wearable structure 320 is worn by the human body, the first electrode 311 and the second electrode 312 may be located on the two sides of the midsagittal plane of the human body, respectively, to detect potentials (e.g., the first potential and the second potential) on the two sides of the midsagittal plane of the human bod, respectively, and the ECG signal may be determined based on the difference between the potentials on the two sides of the midsagittal plane of the human body. In this case, the wearable structure 320 may be worn on a waist region of the human body, such that the first electrode 311 and the second electrode 312 may be located on the two sides of the midsagittal plane of the human body, respectively, i.e., the first electrode 311 and the second electrode 312 may be located on the two sides of the human body, respectively. The difference between the potentials on the sides of the midsagittal plane of the human body can reflect an intensity of the ECG signal. By increasing a spacing between the first electrode 311 and the second electrode 312, the intensity of the ECG signal can be increased. In some embodiments, as shown in FIG. 3, the wearable structure 320 may have a first extension direction and a second extension direction perpendicular to the first extension direction. The first electrode 311 and the second electrode 312 may be spaced apart along the first extension direction of the wearable structure. In some embodiments, the first extension direction of the wearable structure 320 refers to a circumferential direction of the wearable structure 320 worn on the waist region of the human body when the wearable structure 320 is worn on the human body.

In some embodiments, the wearable structure 320 may be an elastic belt structure, and the first electrode 311 and the second electrode 312 may be spaced apart on the elastic belt structure along an extension direction (or a length direction) of the elastic belt structure. The user may fasten the elastic belt structure to the waist region of the user by tying the elastic belt structure or fixing the elastic belt structure with buckles, such that the first electrode 311 and the second electrode 312 may be located on the two sides of the midsagittal plane of the human body, respectively. The first extension direction of the wearable structure 320 may be the extension direction of the elastic belt structure. In some embodiments, the wearable structure 320 may be in the form of a waist belt as shown in FIG. 4, and the first electrode 311 and the second electrode 312 may be spaced apart on a surface where the waist belt fits the human skin. The first extension direction of the wearable structure 320 may be a length direction of the waist belt after the waist belt is unfolded. In some embodiments, two ends of the waist belt may be combined through a touch fastener or a buckle, etc., so as to facilitate the user to wear or take off the waist belt. The wearable structure 320 may be the elastic belt structure, which ensures that the user has good wearing comfort and the wearable device 300 is washable. In some embodiments, the wearable structure 320 may be in the form of trousers (e.g., shorts, trousers, jumpsuits, etc.) shown in FIG. 5, and the first electrode 311 and the second electrode 312 may be arranged at interval along a circumferential direction of a waist of the trousers on an inner side of the waist of the trousers. When the human body wears the trousers, the first electrode 311 and the second electrode 312 disposed at the waist of the trousers may be located on the two sides of the midsagittal plane of the human body, respectively. For example, the first electrode 311 and the second electrode 312 may be located at a left waist side and a right waist side, a left buttock side and a right buttock side, a left knee joint and a right knee joint, a left ankle, and a right ankle(as shown in a dotted rectangular position in FIG. 5), , etc. In some embodiments, the wearable structure 320 may be in the form of a skirt, and the first electrode 311 and the second electrode 312 may be arranged at interval along a circumferential direction of a waist of the skirt on an inner side of the waist of the skirt, such that the first electrode 311 and the second electrode 312 may be located on the two sides of the midsagittal plane of the human body, respectively. The first extension direction of the wearable structure 320 may be a length direction of the waist of the trousers or the waist of the skirt after the trousers or the skirt is unfolded along the circumferential direction.

In some embodiments, the first electrode 311 and the second electrode 312 of the wearable device 300 may also be detachably connected with the wearable structure 320. For example, the wearable structure 320 may be a belt structure or trousers, and a side of the first electrode 311 or the second electrode 312 that does not contact the human skin may be connected with the wearable structure 320 by bonding, snapping, embedding, etc. When the user needs to measure the ECG signal, the first electrode 311 and the second electrode 312 may be disposed on the waist, ends of trouser legs, middle of the trouser legs, and the buttocks of the belt structure or the trousers. Meanwhile, the positions of the first electrode 311 and the second electrode 312 on the wearable structure 320 may be adjusted based on the figure (e.g., a height, a weight, a waist circumference, etc.) of the user to suit users of different figures. When the user needs to wash the wearable structure 320 (e.g., the belt structure or the trousers), the first electrode 311, the second electrode 312, and other components may be removed from the wearable structure 320 to prevent the wearable device 300 from damaging the first electrode 311, the second electrode 312, and other components 312 during the washing process. In some embodiments, a first touch fastener may be provided on a side of the wearable structure 320 (e.g., the belt structure or the waist of trousers) that is attached to the user. The first touch fastener may be arranged along the extension direction of the belt structure or the circumferential direction of the waist of the trousers. A second touch fastener may be provided on a side of the first electrode 311 or the second electrode 312 that is away from a body contact side of the user. The second touch fastener may be bonded to the first touch fastener to achieve a detachable connection between the first electrode 311 and the wearable structure 320, and a detachable connection between the second electrode 312 and the wearable structure 320. In some embodiments, the first touch fastener may cover the side of the wearable structure 320 that is attached to the user. When the first touch fastener is an integral structure, the first touch fastener may affect the elasticity of the wearable structure 320, resulting in a poor wearing experience of the user. In some embodiments, a plurality of first touch fasteners may be provided. The plurality of first touch fasteners may be sequentially spliced on the side of the wearable structure 320 that is attached to the user. In this case, gaps between the plurality of first touch fasteners enable the wearable structure 320 to maintain the elasticity, and are also convenient to adjust the positions of the first electrode 311 and the second electrode 312 on the wearable structure 320, so as to be suitable for users of different figures. In some embodiments, the plurality of first touch fasteners may also be distributed and spaced along the extension direction of the belt structure or the circumferential side of the position of the electrode. For example, a length of each of the plurality of first touch fasteners may be 4 cm, and a spacing between two adjacent first touch fasteners of the plurality of first touch fasteners may be 1 cm. In some embodiments, the plurality of first touch fasteners or second touch fasteners may be made of a non-woven fabric material, which is relatively soft. When the user wears the wearable device 300 and the skin of the user's waist contacts the plurality of first touch fasteners, the plurality of first touch fasteners may not cause discomfort to the user, thereby improving the user experience of the user wearing the wearable device 300. In some embodiments, the wearable device 300 may include a plurality of electrode modules (e.g., the first electrode 311 and the second electrode 312). Each of the plurality of electrode modules may be provided with a wire. One end of the wire may be connected with the first electrode 311 or the second electrode 312 of the electrode module, and the other end of the wire may be provided with an interface plug-in. The interface plug-in may be electrically connected with a circuit structure of the wearable device 300. For example, a port adapted to the interface may be provided on a circuit board of the circuit structure. The user may replace different electrode modules as needed. In some embodiments, sizes of the plurality of electrode modules may be the same or different. Merely by way of example, the size of the first electrode or the second electrode of each of the plurality of electrode modules be 1 cm*1 cm, 1 cm*2 cm, 1 cm*3 cm, 1 cm*4 cm, 1 cm*5 cm, 2 cm*2 cm, 2 cm*3 cm, 2 cm*4 cm, 2 cm*5 cm, 3 cm*3 cm, 3 cm*4 cm, 3 cm*5 cm, 4 cm*4 cm, 4 cm*5 cm, or 5 cm*5 cm. It should be noted that the size of the first electrode or the second electrode of each of the plurality of electrode modules is not limited to the sizes listed above, but may include other sizes.

In some embodiments, the first electrode 311 or the second electrode 312 may be attached to the human skin in the form of a flexible patch. The flexible patch may be a regular shape such as a circle, an ellipse, a rectangle, a diamond, or other irregular shapes. In practical applications, the shape of the first electrode 311 or the second electrode 312 may be set based on a shape of a bone under the skin of the waist region where the first electrode 311 or the second electrode 312 is attached. In some embodiments, the first electrode 311 or the second electrode 312 may be an electrode made of a single material, such as a metal fabric electrode, a conductive silicon electrode, a hydrogel electrode, a metal electrode, etc.

Preferably, the first electrode 311 or the second electrode 312 may be the metal fabric electrode and the conductive silicon electrode. Further preferably, the first electrode 311 or the second electrode 312 may be the metal fabric electrode. The metal fabric electrode has a small resistivity, and an impedance of the metal fabric electrode and a contact impedance between the metal fabric electrode and the skin are also small. The smaller the contact impedance between the first electrode 311 or the second electrode 312 and the skin, the more conducive it is to reduce the interference of movement artifacts on the ECG signal collected by the first electrode 311 and the second electrode 312. In some embodiments, the first electrode 311 or the second electrode 312 may protrude from a surface of the wearable structure 320 around the first electrode 311 or the second electrode 312, such that a pre-pressure may be provided for the first electrode 311 or the second electrode 312, which causes the first electrode 311 or the second electrode 312 to be fully attached to the human skin to accurately collect the ECG signal. In some embodiments, a height of the first electrode 311 or the second electrode 312 protruding relative to the surface of the wearable structure 320 around the first electrode 311 or the second electrode 312 may depend on a thickness of the first electrode 311 or the second electrode 312. The thickness of the first electrode 311 or the second electrode 312 refers to a size of the first electrode 311 or the second electrode 312 in a direction perpendicular to the surface of the wearable structure 320 around the first electrode 311 or the second electrode 312. The thickness of the first electrode 311 or the second electrode 312 may be related to a material of the first electrode 311 or the second electrode 312. For example, in some embodiments, when the ECG signal is collected using the metal fabric electrode, the thickness of the metal fabric electrode may be in a range of 10 $\mu$m-5 mm. Preferably, the thickness of the metal fabric electrode may be in a range of 100 $\mu$m-3 mm. Further preferably, the thickness of the metal fabric electrode may be in a range of 500 $\mu$m-2 mm. In some embodiments, the first electrode 311 or the second electrode 312 may be an electrode formed by superimposing different materials, such as an electrode composed of the metal fabric material and the conductive silicon material. The contact impedance between the electrode and the skin is small, and the conductive silicon in contact with the skin has the advantages of skin-friendliness, washing resistance, and friction resistance, thereby avoiding discomfort to the human body caused by the contact between the electrode and the skin.

In some embodiments, a size of the first electrode 311 or a size of the second electrode 312 in the first extension direction or a size of the first electrode 311 or a size of the second electrode 312 in the second extension direction should be as large as possible, provided that the size of the first electrode 311 or the size of the second electrode 312 in the first extension direction or the size of the first electrode 311 or the size of the second electrode 312 in the second extension direction does not exceed a size of the wearable structure 320 in the first extension direction or a size of the wearable structure 320 in the second extension direction, so as to ensure that the first electrode 311 or the second electrode 312 has a large contact area with the skin, and reduce the contact impedance between the first electrode 311 or the second electrode 312 and the contacted skin, thereby reducing the interference of movement artifacts on the ECG signal. Meanwhile, the large size of the first electrode 311 or the second electrode 312 in the first extension direction or the large size of the first electrode 311 or the second electrode 312 in the second extension direction may cause the first electrode 311 or the second electrode 312 to be less susceptible to deformation and displacement during wearing, which is conducive to reducing the movement artifacts and improving the quality of the ECG signal. In addition, the large size of the first electrode 311 or the second electrode 312 in the first extension direction or the large size of the first electrode 311 or the second electrode 312 in the second extension direction ensures that the first electrode 311 or the second electrode 312 does not completely fall off the skin in various movements or wrinkles, and thus the collection of the ECG signal is not affected. In some embodiments, the size of the first electrode 311 or the second electrode 312 in the first extension direction or the size of the first electrode 311 or the second electrode 312 in the second extension direction refers to a maximum size of the first electrode 311 or the second electrode 312 in the first extension direction or a maximum size of the first electrode 311 or the second electrode 312 in the second extension direction, respectively. In some embodiments, the size of the first electrode 311 or the second electrode 312 in the first extension direction may be in a range of 5 mm-50 mm, and the size of the first electrode 311 or the second electrode 312 in the second extension direction may be in a range of 5 mm-50 mm. In some embodiments, the size of the first electrode 311 or the second electrode 312 in the first extension direction may be in a range of 10 mm-45 mm, and the size of the first electrode 311 or the second electrode 312 in the second extension direction may be in a range of 10 mm-45 mm. In some embodiments, the size of the first electrode 311 or the second electrode 312 in the first extension direction may be in a range of 15 mm-40 mm, and the size of the first electrode 311 or the second electrode 312 in the second extension direction may be in a range of 15 mm-40 mm. In some embodiments, the size of the first electrode 311 or the second electrode 312 in the first extension direction may be in a range of 20 mm-30 mm, and the size of the first electrode 311 or the second electrode 312 in the second extension direction may be in a range of 20 mm-30 mm.

In some embodiments, an isolation layer may be provided between the first electrode 311 or the second electrode 312 and a surface of the wearable structure 320 close to the human skin. Specifically, the isolation layer may be located on a surface of the wearable structure 320 around the first electrode 311 or the second electrode 312. The isolation layer may prevent the wearable structure 320 from accidentally connecting the first electrode 311 or the second electrode 312 when the wearable structure 320 is soaked, resulting in a weak or inaccurate collected ECG signal. In some embodiments, the isolation layer may be made of an insulating and waterproof material. In some embodiments, the material of the isolation layer may include rubber, a high molecular polymer, silicone, or the like, or any combination thereof.

The wearable device 300 provided in the embodiments of the present disclosure can be worn on the waist region, a buttock region, a knee joint region, an ankle region, etc. of the human body, such that the first electrode 311 and the second electrode 312 may be located on two sides of a midsagittal plane of the human body, respectively and attached to the skin of the waist region on the two sides of the midsagittal plane of the human body to collect the ECG signal of the human body. In this way, a heart condition of the user who is wearing the wearable device 300 can be monitored and a good wearing comfort of the user can be ensured. In some embodiments, the contact positions of the first electrode 311 and the second electrode 312 with the waist region on the two sides of the midsagittal plane of the human body may be related to the quality and the intensity of the collected ECG signal. The contact positions of the first electrode 311 and the second electrode 312 with the human skin are described in detail below with reference to a schematic diagram illustrating a human body.

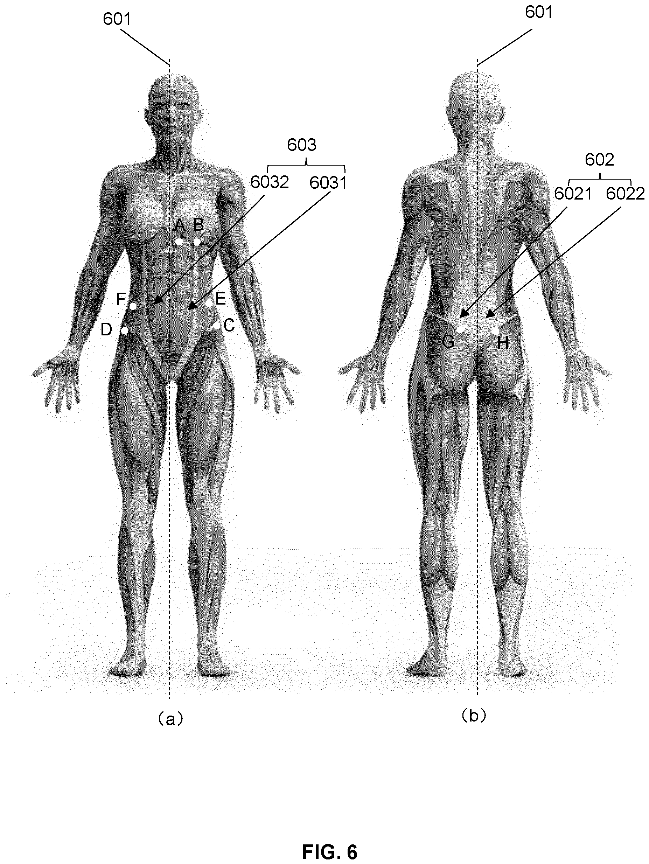

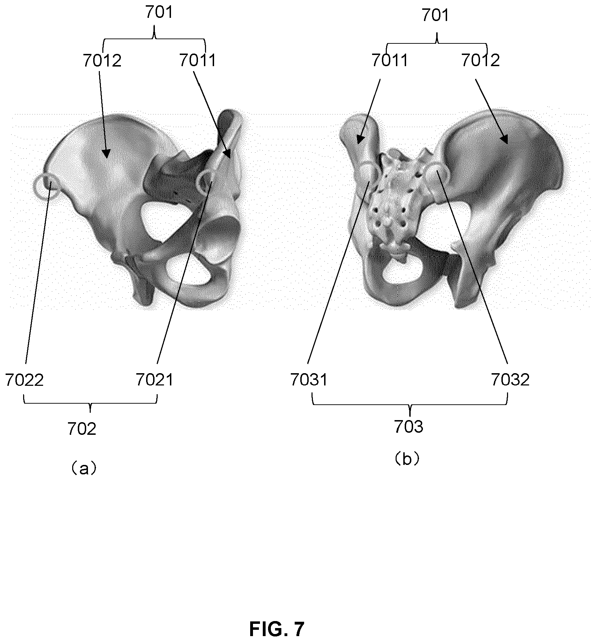

FIG. 6 is a schematic diagram illustrating an exemplary human body. Part (a) in FIG. 6 shows a front side of the human body. Part (b) in FIG. 6 shows a back side of the human body. FIG. 7 is a schematic diagram illustrating an exemplary ilium of a waist region of a human body. Part (a) in FIG. 7 shows a front side of the ilium. Part (b) in FIG. 7 shows a back side of the ilium.

As shown in part (a) and part (b) in FIG. 6, a midsagittal plane of the human body refers to a plane passing through a median line 601 of the human body and dividing the human body into two equal or similar parts. The median line 601 of the human body may be determined based on a connection line from the tip of the nose to the middle of the two nipples of the human body, a connection line from the middle of the two nipples to the middle of the umbilicus of the abdomen, or a connection line from the middle of the umbilicus of the abdomen to the middle of the pubic symphysis joint. The waist region of the human body may be a region between a lower end of the ribs of the human body and a lower end of the ilium. The waist region of the human body may include the abdomen and the ilium. In some embodiments, when the human body wears the wearable structure 320, the first electrode 311 and the second electrode 312 may be attached to a skin of the waist region on two sides of the midsagittal plane of the human body, respectively. The midsagittal plane of the human body divides the waist region of the human body into a left waist region and a right waist region. In some embodiments, the first electrode 311 and the second electrode 312 may be attached to a skin of the left waist region and the right waist region, respectively. In some embodiments, the first electrode 311 and the second electrode 312 may be attached to a skin of a left buttock and a right buttock, respectively. In some embodiments, the first electrode 311 and the second electrode 312 may be attached to a skin of a left knee joint and a right knee joint, respectively. In some embodiments, the first electrode 311 and the second electrode 312 may be attached to a skin of a left ankle and a right ankle, respectively. It is understood that the first electrode 311 and the second electrode 312 may be attached to other parts, such as a skin of a left thigh and a right thigh, a skin of a left calf and a right calf, etc. In some embodiments, the first electrode 311 and the second electrode 312 may be symmetrically arranged with respect to the midsagittal plane of the human body, such that movement artifacts of positions where the first electrode 311 and the second electrode 312 are attached may have good consistency, which is conducive to eliminating the movement artifacts. For example, the movement artifacts in an ECG signal may be eliminated by a differential amplifier circuit to improve the quality of the ECG signal. In some embodiments, by maintaining the consistency of the first electrode 311 and the second electrode 312, the consistency of the movement artifacts of the positions where the first electrode 311 and the second electrode 312 are attached may be further improved, which is more conducive to eliminating the movement artifacts and making the ECG signal have a higher quality. In some embodiments, the consistency of the first electrode 311 and the second electrode 312 may include consistency in a material, a size (e.g., the size in the first extension direction, the size in the second extension direction, the thickness, etc.), a pressure on the skin, or the like, or a combination thereof. In some embodiments, the movement artifacts of the positions where the first electrode 311 and the second electrode 312 are attached may have good consistency by making parts of wearable structure 320 carrying the first electrode 311 and the second electrode 312 elastically consistent, so as to eliminate the movement artifacts and improve the quality of the ECG signal. It should be noted that in some embodiments, the first electrode 311 and the second electrode 312 may be asymmetrically arranged with respect to the midsagittal plane of the human body. For example, when the user wears the wearable device, the first electrode 311 may be located on the abdomen of the human body on one side of the midsagittal plane, and the second electrode 312 may be located on the ilium of the human body on the other side of the midsagittal plane. As another example, when the user wears the wearable device, the first electrode 311 may be located on the abdomen of the human body on one side of the midsagittal plane, and the second electrode 312 may be located on the back waist of the human body on the other side of the midsagittal plane. In addition, the material, the size, the pressure on the skin, or the like, of the first electrode 311 and the second electrode 312 may be consistent or inconsistent.

In some embodiments, as shown in part (a) and part (b) in FIG. 7, when the human body wears the wearable structure 320, the first electrode 311 and the second electrode 312 may be attached to positions of the ilium 701 on the two sides of the midsagittal plane of the human body, respectively. Merely by way of example, the midsagittal plane of the human body divides an ilium 701 into a left ilium 7011 and a right ilium 7012. The first electrode 311 and the second electrode 312 may be attached to skin regions corresponding to the left ilium 7011 and the right ilium 7012, respectively, to collect the ECG signal of the human body. In some embodiments, when the human body wears the wearable structure 320, contact positions of the first electrode 311 and the second electrode 312 with the skin regions covering the left ilium 7011 and the right ilium 7012, respectively, may be symmetrical with respect to the midsagittal plane of the human body, such that the consistency of the movement artifacts of the contact positions of the first electrode 311 and the second electrode 312 can be improved, thereby eliminating the movement artifacts and improving the quality of the ECG signal. In some embodiments, the contact positions of the first electrode 311 and the second electrode 312 with the skin regions covering the left ilium 7011 and the right ilium 7012, respectively, may be asymmetrical with respect to the midsagittal plane of the human body.

In some embodiments, as shown in part (a) in FIG. 7, when the human body wears the wearable structure 320, the first electrode 311 and the second electrode 312 may be attached to positions of anterior superior iliac spines 702 on the two sides of the midsagittal plane of the human body, respectively. The anterior superior iliac spines 702 may include a left anterior superior iliac spine 7021 and a right anterior superior iliac spine 7022 located on the left ilium 7011 and the right ilium 7012, respectively. Merely by way of example, when the human body wears the wearable structure 320, the first electrode 311 and the second electrode 312 may be attached to skin regions corresponding to the left anterior superior iliac spine 7021 and the right anterior superior iliac spine 7022, respectively, to collect the ECG signal of the human body. Since there are fewer muscles at the left anterior superior iliac spine 7021 and the right anterior superior iliac spine 7022, even when the human body is in a movement state, an electromyographic signal has less interference with the ECG signal collected by the first electrode 311 and the second electrode 312, which ensures that the ECG signal has a good quality. The left anterior superior iliac spine 7021 and the right anterior superior iliac spine 7022 are symmetrical with respect to the midsagittal plane of the human body. The movement artifacts corresponding to the first electrode 311 and the second electrode 312 may have good consistency, which is conducive to eliminating the movement artifacts and improving the quality of the ECG signal. In addition, a difference between a distance from the left anterior superior iliac spine 7021 to the heart and a distance from the right anterior superior iliac spine 7022 to the heart is large, and a difference between a potential between the left anterior superior iliac spine 7021 and the heart and a potential between the right anterior superior iliac spine 7022 and the heart is also large. The first electrode 311 and the second electrode 312 may be attached to the skin regions corresponding to the left ilium 7011 and the right ilium 7012, respectively, which can improve the intensity of the collected ECG signal. In addition, the left anterior superior iliac spine 7021 and the right anterior superior iliac spine 7022 have protrusions so as to be fully attached to the first electrode 311 and the second electrode 312, ensuring that the first electrode 311 and the second electrode 312 are not liable to fall off. The human body is less sensitive to the skin regions corresponding to the left anterior superior iliac spine 7021 and the right anterior superior iliac spine 7022, respectively, which ensures that the human body has a good wearing comfort when wearing the wearing structure 320.

In some embodiments, as shown in part (b) in FIG. 7, the first electrode 311 and the second electrode 312 may be attached to posterior superior iliac spines 703 on the two sides of the midsagittal plane of the human body. The posterior superior iliac spines 703 may include a left posterior superior iliac spine 7031 located on the left ilium 7011 and a right posterior superior iliac spine 7032 located on the right ilium 7012. Merely by way of example, when the human body wears the wearable structure 320, the first electrode 311 and the second electrode 312 may be attached to skin regions corresponding to the left posterior superior iliac spine 7031 and the right posterior superior iliac spine 7032, respectively, to collect the ECG signal of the human body. Since there are fewer muscles at the left posterior superior iliac spine 7031 and the right posterior superior iliac spine 732, even when the human body is in the movement state, the electromyographic signal has less interference with the ECG signal collected by the first electrode 311 and the second electrode 312, which ensures that the ECG signal has good quality. In addition, the left posterior superior iliac spine 7031 and the right posterior superior iliac spine 7032 are symmetrical with respect to the midsagittal plane of the human body, and the movement artifacts have good consistency, which is conducive to eliminating the movement artifacts and improving the quality of the ECG signal.

In some embodiments, as shown in part (b) in FIG. 6, when the human body wears the wearable structure 320, the first electrode 311 and the second electrode 312 may be attached to positions of a back waist 602 on the two sides of the midsagittal plane of the human body. Merely by way of example, the midsagittal plane of the human body divides the back waist 602 into a left back waist 6021 and a right back waist 6022. When the human body wears the wearable structure 320, the first electrode 311 and the second electrode 312 may be attached to skin regions corresponding to the left back waist 6021 and the right back waist 6022, respectively, to collect the ECG signal. In some embodiments, when the human body wears the wearable structure 320, contact positions of the first electrode 311 and the second electrode 312 with the skin regions of the left back waist 6021 and the right back waist 6022, respectively, may be symmetrical about the midsagittal plane of the human body, such that the consistency of the movement artifacts of the contact positions of the first electrode 311 and the second electrode 312 can be improved, thereby eliminating the movement artifacts and improving the quality of the ECG signal. In some embodiments, the contact positions of the first electrode 311 and the second electrode 312 with the skin regions of the left back waist 6021 and the right back waist 6022, respectively, may be asymmetrical with respect to the midsagittal plane of the human body.

In some embodiments, as shown in part (a) in FIG. 6, when the human body wears the wearable structure 320, the first electrode 311 and the second electrode 312 may be attached to an abdomen 603 on the two sides of the midsagittal plane of the human body, respectively. Merely by way of example, the midsagittal plane of the human body divides the abdomen 603 into a left abdomen 6031 and a right abdomen 6032. When the human body wears the wearable structure 320, the first electrode 311 and the second electrode 312 may be attached to skin regions of the left abdomen 6031 and the right abdomen 6032, respectively, to collect the ECG signal. In some embodiments, when the human body wears the wearable structure 320, contact positions of the first electrode 311 and the second electrode 312 with the skin regions of the left abdomen 6031 and the right abdomen 6032 may be symmetrical with respect to the midsagittal plane of the human body, which can improve the consistency of the movement artifacts of the contact positions of the first electrode 311 and the second electrode 312, thereby eliminating the movement artifacts and improving the quality of the ECG signal. In some embodiments, the contact positions of the first electrode 311 and the second electrode 312 with the skin regions of the left abdomen 6031 and the right abdomen 6032 may be asymmetrical with respect to the midsagittal plane of the human body.

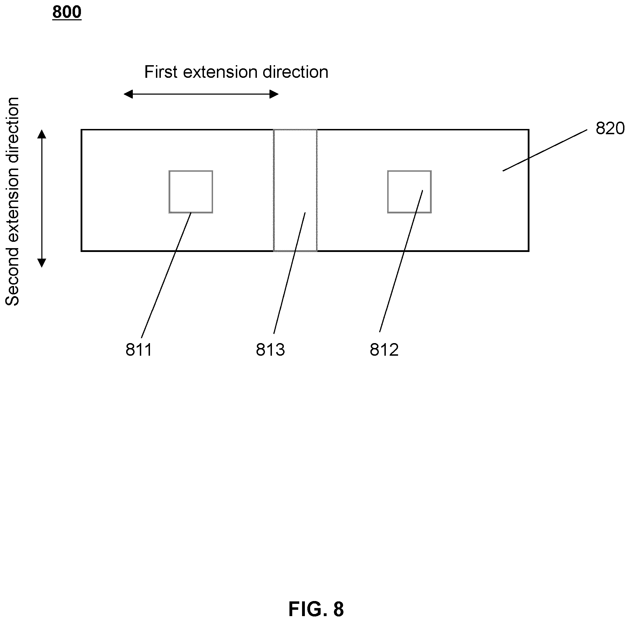

FIG. 8 is a schematic structural diagram illustrating an exemplary wearable device according to some embodiments of the present disclosure.

In some embodiments, as shown in FIG. 8, the wearable device 800 may include a first electrode 811, a second electrode 812, a reference electrode 813, and a wearable structure 820. The first electrode 811 and the second electrode 812 may be disposed on a surface of the wearable structure 820 close to a human skin at interval. The reference electrode 813 may be disposed between the first electrode 811 and the second electrode 812. The first electrode 811, the second electrode 812, and the wearable structure 820 may be similar to the first electrode 311, the second electrode 312, and the wearable structure 320 of the wearable device 300. More descriptions regarding the first electrode 811, the second electrode 812, and the wearable structure 820 may be found in the related descriptions of the first electrode 311, the second electrode 312, and the wearable structure 320, which are not repeated here.

In some embodiments, when the human body wears the wearable structure 820, the first electrode 811 and the second electrode 812 may be attached to two sides of a midsagittal plane of the human body, respectively, and the reference electrode 813 may be attached to a skin of the human body. In some embodiments, the reference electrode 813 may provide a reference ground voltage for the first electrode 811 and the second electrode 812, so as to process a first potential corresponding to the first electrode 811 and a second potential corresponding to the second electrode 812 using a circuit (e.g., a differential amplifier) subsequently. In some embodiments, the reference electrode 813 may also be electrically connected with a right leg drive circuit. The right leg drive circuit may be configured to reduce an impedance of the reference electrode 813 to the human body, such that the right leg drive circuit can more effectively reduce the power frequency interference. In some embodiments, when the human body wears the wearable device 800, the reference electrode 813 may be located at other positions such as a back waist side of the waist region, and the abdomen of the human body. Preferably, when the human body wears the wearable device 800, the first electrode 811 and the second electrode 812 may be symmetrically arranged with respect to the midsagittal plane of the human body. Accordingly, the reference electrode 813 may be located in a center (e.g., the lumbar spine and its vicinity) of the back waist side of the waist region of the human body, such that when the user wears the wearable device 800, each electrode may be symmetrically arranged with respect to the midsagittal plane of the human body, a value of a potential difference between the first potential corresponding to the first electrode 811 and a potential corresponding to the reference electrode 813 may be approximately equal to a value of a potential difference between the second potential corresponding to the second electrode 812 and the potential corresponding to the reference electrode 813, thereby facilitating the subsequent processing of the circuit (e.g., the differential amplifier). In addition, there are fewer muscles on the back waist side of the human body, the influence of the electromyographic signal on the ECG signal is relatively small, and the back waist side of the human body has a low sensitivity to an external object or simulation, which ensures that the user has a more comfortable wearing experience when using the wearable device 800. It should be noted that a count of the reference electrode 813 is not limited to one reference electrode 813 shown in FIG. 8, and a plurality of reference electrodes 813 may be provided. For example, the reference electrode 813 may include a first reference electrode and a second reference electrode. When the human body wears the wearable device 800, the first reference electrode and the second reference electrode may be located at left and right rear waists of the waist region of the human body, respectively. The first reference electrode and the second reference electrode may be symmetrically arranged with respect to the midsagittal plane of the human body, such that when the human body wears the wearable device 800, the electrodes disposed on the wearable device 800 may be symmetrically arranged with respect to the midsagittal plane of the human body. In some embodiments, the first reference electrode and the second reference electrode may be arranged asymmetrically with respect to the midsagittal plane of the human body. For example, the first reference electrode and the second reference electrode may be both located at the left rear waist or the right rear waist of the human body. As another example, the first reference electrode may be located at a position of the left rear waist of the human body away from the midsagittal plane of the human body, and the second reference electrode may be located at a position of the right rear waist of the human body close to the midsagittal plane of the human body. In some embodiments, the first reference electrode and the second reference electrode may be connected by a wire such that the first reference electrode and the second reference electrode are at the same potential. In this case, the first reference electrode and the second reference electrode may be used as one reference electrode, thereby increasing the degree of freedom of the wearable device 800 with respect to the symmetry setting. In addition, the first reference electrode and the second reference electrode may not be limited to being disposed on the back waist side of the waist region of the human body, but may also be located in other regions such as the abdomen, left and right waist sides, left and right buttocks, left and right thighs, etc.

In some embodiments, a size of the reference electrode 813 in a second extension direction of the wearable structure 820 may not be less than a size of the first electrode 811 or the second electrode 812 in the second extension direction of the wearable structure 820, such that the reference electrode 813 may be attached to the human body. In some embodiments, the size of the reference electrode 813 in the second extension direction may be in a range of 5 mm-80 mm. In some embodiments, the size of the reference electrode 813 in the second extension direction may be in a range of 10 mm-80 mm. In some embodiments, the size of the reference electrode 813 in the second extension direction may be in a range of 20 mm-80 mm. In some embodiments, the size of the reference electrode 813 in the second extension direction may be in a range of 30 mm-80 mm. It should be noted that the size of the reference electrode 813 in the second extension direction of the wearable structure 820 may be less than the size of the first electrode 811 or the second electrode 812 in the second extension direction of the wearable structure 820, as long as the reference ground voltage can be provided.

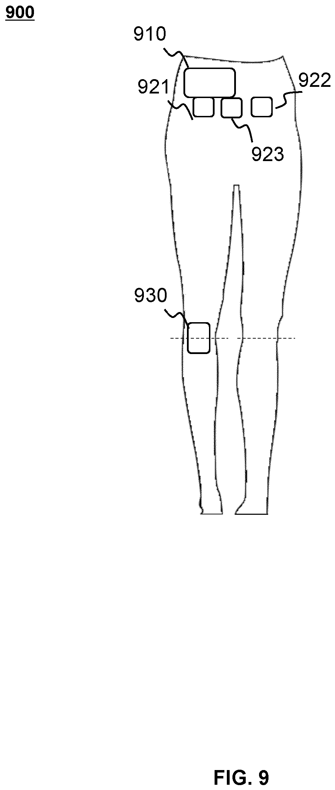

FIG. 9 is a schematic structural diagram illustrating a front side of trousers according to some embodiments of the present disclosure. As shown in FIG. 9, a wearable device may be trousers 900. The trousers 900 may include a first strain sensor 910, a first electrode 921, a second electrode 922, a reference electrode 923, and a second strain sensor 930.

In some embodiments, the first strain sensor 910 may be disposed on a wearable structure and attached to a waist region of a human body. The first strain sensor 910 may be configured to collect respiratory state information of the human body based on a fluctuation of the waist region when the human body breathes.

In some embodiments, the first strain sensor 910 may be a belt/ring structure around a waist of a user. For example, the first strain sensor 910 may be a belt sensor that is elastic and capable of extending around the waist. When the user wears the trousers 900 and performs an inhalation exercise, a circumference of the thorax expands and the diaphragmatic dome drops, causing a circumference of the first strain sensor 910 to change, and the first strain sensor 910 may be configured to measure the change of the circumference. Similarly, when the user wears the trousers 900 and performs an exhalation exercise, the circumference of the thorax shrinks and the diaphragmatic dome rises, and the first strain sensor 910 may be configured to measure the change of the circumference change at this time. The process of the change of the circumference during the inhalation exercise and the exhalation exercise may be regarded as a breathing process, thereby obtaining the respiratory state information of the human body. In some embodiments, the first strain sensor 910 may be disposed not around the waist of the human body. For example, the first strain sensor 910 may be attached to an abdominal region of the human body. When the human body breathes, the abdominal region may fluctuate, and the first strain sensor 910 may deform based on the fluctuation of the abdominal region, thereby obtaining the respiratory state information of the user. In some embodiments, one or more first strain sensors 910 may be provided. When a plurality of first strain sensors 910 are provided, the plurality of first strain sensors 910 may be attached to different regions of the abdominal region, thereby obtaining more accurate respiratory state information. In some embodiments, the first strain sensor 910 may include any one or more of a strain pressure sensor, a strain torque sensor, a strain displacement sensor, a strain acceleration sensor, etc. In some embodiments, the first strain sensor 910 may be replaced by an air pressure sensor. For example, the air pressure sensor may include a closed flexible cavity and a pressure sensing element. The air pressure sensor may be sealed and connected with the flexible cavity. When the human body wears the air pressure sensor, the air pressure sensor may be attached to the waist region or the abdomen of the human body. When the human body breathes, the fluctuation of the waist region or the abdomen may act on the flexible cavity of the air pressure sensor, and an internal pressure of the flexible cavity may change. The pressure sensing element may convert a pressure intensity change into an electrical signal to obtain a fluctuation change in the waist region or the abdomen when the human body breathes, thereby determining the respiratory state information of the human body. In some embodiments, one or more air pressure sensors may be provided. When a plurality of air pressure sensors are provided, the plurality of air pressure sensors may be distributed at different positions of the waist region or the abdomen of the human body to obtain the fluctuation of different positions of the waist region or the abdomen.

The respiratory state information can reflect a physiological structure change of the human body during the breathing process. In some embodiments, the respiratory state information of the human body may include a breathing frequency, a breathing depth, an inhalation time, an exhalation time, and a breathing smoothness. The breathing frequency, the inhalation time, and the exhalation time may be determined by measuring the time of the change of the circumference. The breathing depth may be determined by measuring a peak value (e.g., a maximum circumference and a minimum circumference) of the change of the circumference. The breathing smoothness may be determined by measuring a plurality of breathing processes and comparing the breathing depth, the inhalation time, and the exhalation time of the plurality of breathing processes. Real-time monitoring of an exhalation process may be achieved through the first strain sensor 910.

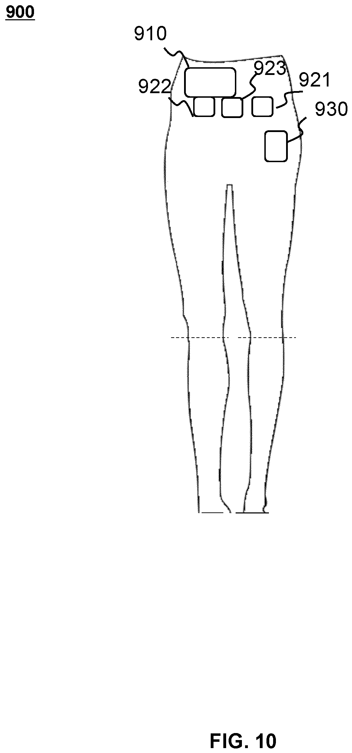

The descriptions regarding the first electrode 921 and the second electrode 922 may be found in the related descriptions of the first electrode 311 and the second electrode 312 shown in FIG. 3, FIG. 4, and FIG. 5, or may be found in the related descriptions of the first electrode 811 and the second electrode 812 shown in FIG. 8. In some embodiments, at least two electrodes (the first electrode 921 and the second electrode 922) may be disposed on the wearable structure at interval. When the human body wears wearable structure trousers 900, the at least two electrodes (the first electrode 921 and the second electrode 922) may be located on two sides of the midsagittal plane of the human body, i.e., the first electrode 921 may be located on a left side of the midsagittal plane of the human body, and the second electrode 922 may be located on a right side of the midsagittal plane of the human body. For example, the first electrode 921 may be located on a left leg side of the midsagittal plane of the human body, and the second electrode 922 may be located on a right leg side of the midsagittal plane of the human body. Preferably, when the human body wears the wearable structure trousers 900, the at least two electrodes (the first electrode 921 and the second electrode 922) may be located on the two sides of the midsagittal plane of the waist region of the human body. For example, the first electrode 921 may be located on a left side of the midsagittal plane of the waist region of the human body, and the second electrode 922 may be located on a right side of the midsagittal plane of the waist region of the human body. In order to be closely attached to the human skin, the first electrode 921 and the second electrode 922 may be located on an inner side of the trousers 900, i.e., a side of the trousers 900 close to the human skin. In some embodiments, the first electrode 921 and the second electrode 922 may be connected with the trousers 900 by a touch fastener, an adhesive, a buckle, a mesh bag, etc. The first electrode 921 and the second electrode 922 may be configured to obtain an ECG signal. In order to reduce the interference of an electromyographic signal, in some embodiments, when the human body wears the wearable structure trousers 900, the first electrode 921 and the second electrode 922 may be attached to an ilium, a back waist, or an abdomen on the two sides of the midsagittal plane of the human body, respectively. The first electrode 921 and the second electrode 922 may be attached to the back waist on the two sides of the midsagittal plane of the human body, as shown in FIG. 10.

In some embodiments, when the human body wears the wearable structure trousers 900, the first electrode 921 and the second electrode 922 may be symmetrically arranged with respect to the midsagittal plane of the human body. The symmetrical arrangement of the electrodes can make movement artifacts of contact positions of the first electrode 921 and the second electrode 922 have a good consistency, which is conducive to eliminating the movement artifacts. For example, the movement artifacts in the ECG signal may be eliminated by a differential amplifier circuit to improve the quality of the ECG signal.

The reference electrode 923 may be configured to provide a reference ground voltage. In some embodiments, the first electrode 921 and the second electrode 922 may be disposed on a surface of the wearable structure trousers 900 close to a human skin at interval, and the reference electrode 923 may be located between the first electrode 921 and the second electrode 922. The reference electrode 923 may be located on an inner side of the trousers 900. Descriptions regarding the reference electrode 923 may be found in the related descriptions of the reference electrode 813 shown in FIG. 8.

The second strain sensor 930 may be configured to obtain a lower limb movement parameter of the human body. The second strain sensor 930 may sense bending and stretching movements of a human joint. Exemplary lower limb movement parameters may include a joint bending angle, a bending direction, etc. In some embodiments, the second strain sensor 930 may be located at a position of the wearable structural trousers 900 corresponding to a leg or a buttock of the human body. FIG. 9 only depicts that the second strain sensor 930 is disposed at an outer side of a left knee joint. It is understood that the second strain sensor 930 may be located at any joint of the lower limbs of the human body, such as a right knee joint, an ankle joint, a hip joint, etc. The second strain sensor 930 may be located at any position of any joint of the lower limbs of the human body, such as an outer side of a left knee joint, an inner side of the left knee joint, etc. The second strain sensor 930 may be located at the position of the wearable structural trousers 900 corresponding to the buttock of the human body, as shown in FIG. 10. In some embodiments, a plurality of second strain sensors 930 may be provided. The plurality of second strain sensors 930 may be located at different lower limb joints.

In some embodiments, a processor may be further configured to recognize an exercise movement of the lower limbs of the human body based on the second strain sensor 930. For example, when the second strain sensor 930 obtains that the knee joint bending angle is 180° and the hip joint bending angle is 120°, the processor may determine that the exercise movement of the lower limbs is squatting; when the second strain sensor 930 obtains that the knee joint bending angle is 0° and the hip joint bending angle is 0°, the processor may determine that the exercise movement of the lower limbs is standing. The second strain sensor 930 can realize real-time monitoring of the exercise movement of the lower limbs of the human body.

FIG. 10 is a schematic structural diagram illustrating a back side of trousers according to some embodiments of the present disclosure.

As shown in FIG. 10, the first strain sensor 910 may be attached to a waist region of a human body. The first electrode 921 and the second electrode 922 may be attached to a back waist on two sides of a midsagittal plane of the human body. The first electrode 921 may be attached to a right back waist on the two sides of the midsagittal plane of the human body, and the second electrode 922 may be attached to a left back waist on the two sides of the midsagittal plane of the human body. The second strain sensor 930 may be located at a position of the wearable structure trousers 900 corresponding to a buttock (e.g., a right buttock) of the human body. It is understood that the position of the above components is only for illustration. Under the same principle, the above components can be located at any position of the lower limbs of the human body. For example, in addition to being disposed at the waist position, the first electrode 921 and the second electrode 922 may also be disposed at the thigh, the calf, the knee joint, the ankle, and other positions.

FIG. 11 is a schematic diagram illustrating a standard electrocardiogram according to some embodiments of the present disclosure. A waveform 1101 shown in FIG. 11 shows features such as a P wave, a QRS complex, an ST segment, a T wave, a P-R interval, a U wave, etc.

In some embodiments, a processor may determine an HRV and a heart rate of a human body based on an ECG signal. The ECG signal obtained by at least two electrodes may be shown in FIG. 11. In some embodiments, the heart rate may be expressed as a count of R waves appearing per unit time (e.g., one minute). For example, the heart rate may be calculated by dividing 60 by a P-P interval or 60 by an R-R interval.

In some embodiments, the processor may determine the HRV by calculating one or more mathematical statistical indicators related to the R-R interval through statistical analysis. The HRV may be presented as statistical indicators such as *SDNN, SDANN, RMSSD, pNN*50, *SDNNi*, and so on. For example, the HRV may be calculated by the following formula (1):

$$SDNN = \sqrt{\frac{1}{N}\sum_{i=1}^{N}\left(RRs_i - \frac{1}{N}\sum_{i=1}^{N}RRs_i\right)^2} \qquad (1)$$

wherein *SDNN* denotes a standard deviation of intervals between adjacent heartbeats; N denotes a count; $RRs_i$ denotes the R-R interval; i denotes a different R-R interval; *SDNN* it is capable of reflecting a total tension of sympathetic and parasympathetic nerves. The normal value of *SDNN* is in a range of 100 milliseconds-150 milliseconds. The abnormal value of *SDNN* is less than 50 milliseconds.

As another example, the HRV may be calculated by the following formula (2) and formula (3):

$$\overline{RR_{s5}} = \frac{1}{N}\sum_{i=1}^{N}RRs_i \qquad (2)$$

$$SDANN = \sqrt{\frac{1}{N}\sum_{i=1}^{N}\left(RR_{s5} - \frac{1}{N}\sum_{i=1}^{N}RR_{s5}\right)^2} \qquad (3)$$

*SDANN* denotes a standard deviation of several intervals after calculating an average value of the R-R interval every 5 min; $RR_{s5}$ denotes an average value of the R-R interval within 5 min; *SDANN* denotes a magnitude of a sympathetic nerve tension, and is related to a slow change component of the heart rate. When the sympathetic nerve tension increases, the value of *SDANN* decreases. The normal value of *SDANN* is in a range of 80 milliseconds-140 milliseconds. The abnormal value of *SDANN* is less than 50 milliseconds.

As another example, the HRV may be calculated by the following formula (4):

$$RMSSD = \sqrt{\frac{1}{N}\sum_{i=1}^{N-1}(RRs_{i+1} - RRs_{i-1})^2} \qquad (4)$$

wherein *RMSSD* denotes a root mean square (RMS) value of the intervals between the adjacent heartbeats. *RMSSD* is capable of reflecting a parasympathetic nerve tension and is related to a rapid change component of the heart rate. When the parasympathetic nerve tension decreases, the value of *RMSSD* decreases. The normal value of *RMSSD* is in a range of 15 milliseconds-45 milliseconds. The abnormal value of *RMSSD* is less than 15 milliseconds.

As another example, the HRV may be calculated by the following formula (5):

$$pNN50 = \frac{num[(RRs_{i+1} - RRs_i) > 50ms]}{N-1} \qquad (5)$$

wherein *pNN50* denotes a proportion that a difference between the intervals of the adjacent heartbeats exceeds 50 milliseconds. *pNN50* It reflects the parasympathetic nerve tension and is related to the rapid change component of the heart rate. When the parasympathetic nerve tension decreases, the value of *pNN50* decreases. The normal value of *pNN50* is in a range of 1%-12%, and the abnormal value of *pNN50* is less than 0.75%.

As another example, the HRV may be calculated by the following formula (6) and formula (7):

$$SDNNs_5 = \sqrt{\frac{1}{N}\sum_{i=1}^{N}\left(RRs_i - \frac{1}{N}\sum_{i=1}^{N}RRs_i\right)^2} \qquad (6)$$

$$SDNNi = \frac{1}{N}\sum_{i=1}^{N}SDNNs_5 \qquad (7)$$

wherein $SDNNi$ denotes an average value of the standard deviation of the intervals of the heartbeats every 5 min; $SDNNs_5$ denotes the standard deviation of the intervals of the heartbeats every 5 min. $SDNNi$ It reflects the sympathetic nerve tension and is related to the slow change component of the heart rate. When the sympathetic nerve tension increases, the value of $SDNNi$ decreases. The normal value of $SDNNi$ is in a range of 40 milliseconds-80 milliseconds. The abnormal value of $SDNNi$ is less than 20 milliseconds. It is understood that the mathematical statistical indicators for determining the HRV may be other mathematical statistical indicators, which are not limited in the present disclosure.

In some embodiments, the processor may analyze a distribution of the R-R intervals in the ECG signal to obtain a variability of the R-R intervals as the HRV. For example, the variability of the R-R intervals may be obtained as the HRV through an R-R interval histogram, an R-R interval difference histogram, a time series diagram of HRV, etc.

In some embodiments, the processor may determine the HRV by frequency domain analysis. For example, the overall HRV may be evaluated by summing the power of all frequency ranges of all normal heartbeat intervals, the power of a low frequency range representing the activity of the sympathetic and parasympathetic nerves, and the power of a high frequency range representing the activity of the parasympathetic nerves, etc.

In some embodiments, the processor may determine the HRV by a nonlinear analysis method. For example, the processor may determine the HRV by analyzing a fractal dimension (a correlation dimension, a Hausdorff dimension, or an information dimension), complexity analysis, a Lyapunov index, a Kolmogorov entropy, approximate entropy analysis, etc.

In some embodiments, the processor may determine the HRV through an electrocardiogram scatter plot, such as a vector length index, a vector angle index, etc.

In some embodiments, the processor may establish a corresponding mathematical model to determine the HRV using a specific mathematical algorithm. For example, the processor may determine the HRV through parameters such as the fractal dimension, a measure entropy, Lyapunov index, complexity, etc.

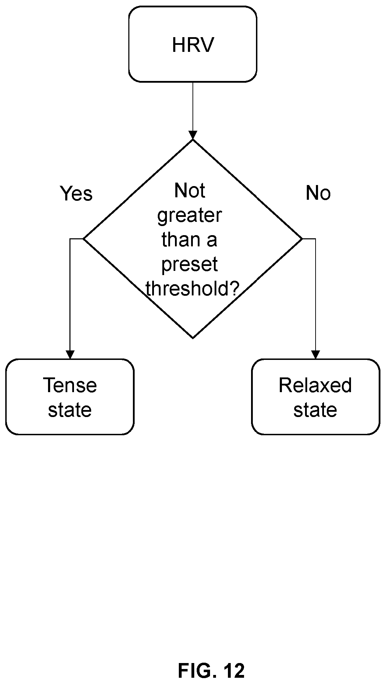

FIG. 12 is a schematic diagram illustrating an exemplary process of determining a physical state of a human body based on an HRV according to some embodiments of the present disclosure.

In some embodiments, the physical state may include a relaxed state and a tense state. The relaxed state refers to a physiological state of the human body when the human body is awake, quiet, emotionally calm, and has a slight amount of exercise/ no exercise. Merely by way of example, the relaxed state may be manifested as a resting heart rate of 60-80 beats /min; mathematical statistical indicators related to the HRV are normal values (greater than a preset threshold). The tense state refers to a physiological state of the human body when the human body is anxious, emotionally unstable, or performing quantitative exercise. The tense state may be manifested as a heart rate of 80-100 beats /minute; the mathematical statistical indicators related to the HRV are abnormal values (not greater than the preset threshold). When the human body is in the relaxed state, a difference between time intervals of heartbeat may be large, which is manifested as a large HRV. When the human body is in a highly tense state, a difference between the time intervals of the heartbeat may be small, which is manifested as a small HRV. In some embodiments, the processor may determine that the physical state of the human body during exercise is the tense state when the HRV is not greater than the preset threshold; determine that the physical state of the human body during exercise is the relaxed state when the HRV is greater than the preset threshold. The preset threshold of the HRV may be determined by an empirical value. If the mathematical statistical indicator of the HRV is $SDNN$, the preset threshold of the HRV may be 50 milliseconds.

In some embodiments, the processor may use at least the HRV as input data into a trained machine learning model, and the trained machine learning model may output the physical state of the human body based on the input data. Exemplary machine learning models may include a deep neural network model, a convolutional neural network model, or the like. In some embodiments, the machine learning model may be obtained by training a large number of training samples with labels. Specifically, a plurality of training samples with labels may be input into an initial model, a loss function may be constructed based on an output of the initial model and the labels, and parameters of the model may be iteratively updated through training based on the loss function. In some embodiments, training may be performed based on the plurality of training samples in various ways. For example, training may be performed based on a gradient descent method. When a preset condition is met, the training may be ended and the trained model may be obtained. The preset condition may be that the loss function converges.

In some embodiments, the plurality of training samples may include a plurality of historical HRV data (e.g., $SDNN$, $SDANN$, $RMSSD$, $pNN$50, $SDNNi$, and other indicators). The indicators may be the physical state of the human body corresponding to each of the plurality of historical HRV data. The machine learning model can realize intelligent recognition of the physical state of the human body, improve recognition efficiency, and reduce the error of the judgment result caused by subjective determination of the physical state.

In some embodiments, the processor may obtain a calibration curve, and determine the physical state of the human body based on the calibration curve. The calibration curve may be obtained by fitting the HRV generated when the human body is in different physical states. For example, the calibration curve may be obtained by fitting the mathematical statistical indicators of HRV *SDNN, SDANN, RMSSD, pNN*50, *SDNNi*, or the like. In some embodiments, an abscissa of the calibration curve may represent time, and an ordinate of the calibration curve may represent a value of each mathematical statistical indicator of HRV. In some embodiments, the processor may use a curve segment of the calibration curve that meets a normal value range as an interval of the relaxed state, and a curve segment of the calibration curve that does not meet the normal value range as an interval of the tense state. For example, for the mathematical statistical indicator *SDNN,* a curve segment of the calibration curve that meets the value of *SDNN* of being in a range of 100 milliseconds-150 milliseconds may be used as the interval of the relaxed state, and a curve segment of the calibration curve that meets the value of *SDNN* of being less than 50 milliseconds may be used as the interval of the tense state.

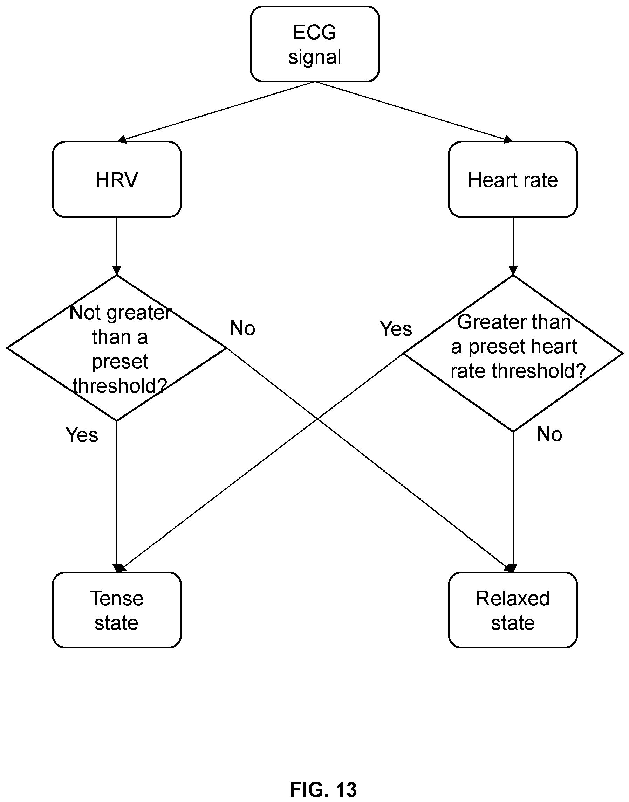

FIG. 13 is a schematic diagram illustrating an exemplary process of determining a physical state of a human body based on an HRV and a heart rate according to some embodiments of the present disclosure.

The heart rate can also reflect the physical state of the human body. In order to further improve the determination accuracy of the physical state of the human body by the wearable device, in some embodiments, the processor may determine the physical state of the human body based on the HRV and the heart rate.

In some embodiments, the processor may determine that the physical state of the human body during exercise is a tense state in response to determining that the HRV is not greater than a preset threshold and the heart rate is greater than a preset heart rate threshold; and determine that the physical state of the human body during exercise is a relaxed state in response to determining that the HRV is greater than the preset threshold and the heart rate is not greater than the preset heart rate threshold. The preset threshold of the HRV may be determined by an empirical value. For example, in response to determining that a mathematical statistical indicator of the HRV is *SDNN,* the preset threshold of the HRV may be 50 milliseconds. The preset heart rate threshold of the heart rate may be set by an empirical value or a physical health condition of a user. For example, the preset heart rate threshold may be 60 times /min, 70 times /min, 80 times/min, etc.

In some embodiments, the processor may use at least the HRV and the heart rate as input data into a trained machine learning model, and the trained machine learning model may output the physical state of the human body based on the input data. Exemplary machine learning models may include a deep neural network model, a convolutional neural network model, or the like. In some embodiments, the machine learning model may be obtained by training a large number of training samples with labels. Specifically, a plurality of training samples with labels may be input into an initial model, a loss function may be constructed based on an output of the initial model and the labels, and parameters of the model may be iteratively updated through training based on the loss function. In some embodiments, training may be performed based on the plurality of training samples in various ways. For example, training may be performed based on a gradient descent method. When a preset condition is met, the training may be ended and the trained model may be obtained. The preset condition may be that the loss function converges.

In some embodiments, the plurality of training samples may include a plurality of historical HRV data (e.g., *SDNN, SDANN, RMSSD, pNN*50, *SDNNi*, and other indicators) and a plurality of historical heart rate data. The indicator may be the physical state of the human body corresponding to each of the plurality of historical HRV data and each of the plurality of historical heart rate data. The machine learning model can realize intelligent recognition of the physical state of the human body, improve the recognition efficiency, and reduce the error of the determination result caused by subjective determination of the physical state. In addition, adding the heart rate data to the model input increases the calculation circumference of the model, thereby improving the degree of fitting with an actual physiological condition.

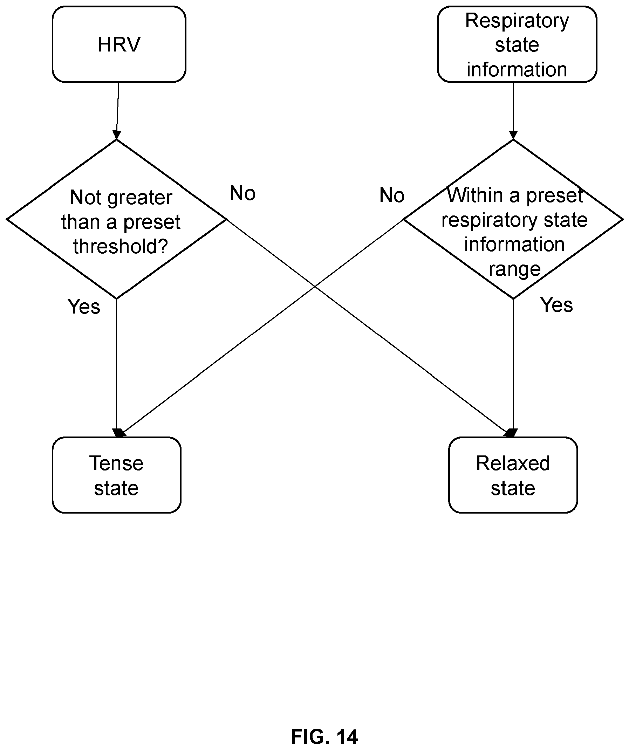

FIG. 14 is a schematic diagram illustrating an exemplary process of determining a physical state of a human body based on an HRV and respiratory state information according to some embodiments of the present disclosure.

Breathing can also reflect the physical state of the human body. In order to further improve the determination accuracy of the physical state of the human body by the wearable device, in some embodiments, the processor may determine the physical state of the human body based on the HRV and the respiratory state information.

In some embodiments, the processor may determine that the physical state of the human body during exercise is a relaxed state in response to determining that the HRV is greater than a preset threshold and the respiratory state information is within a preset respiratory state information range; and determine that the physical state of the human body during exercise is a tense state in response to determining that the HRV is not greater than the preset threshold and the respiratory state information is not within the preset respiratory state information range. The preset threshold of the HRV may be determined by an empirical value. For example, in response to determining that a mathematical statistical indicator of the HRV is *SDNN,* the preset threshold of the HRV may be 50 milliseconds. Merely by way of example, the respiratory state information may include a breathing frequency, a breathing depth, an inhalation time, an exhalation time, and a breathing smoothness. The respiratory state information can reflect the physical state of the human body. For example, in response to determining that the breathing frequency of the human body is normal, the breathing depth is relatively large, the inhalation time and the exhalation time are long, and a breathing process is relatively smooth, it is determined that the human body is in a relaxed state; in response to determining that the breathing frequency of the human body is abnormal (e.g., the breathing frequency is too fast), the breathing depth is relatively small, the inhalation time and the exhalation time are short, and the breathing process is non-smooth, it is determined that the human body is in a tense state. The preset respiratory state information range may be determined by an empirical value. For example, if the respiratory state information is the breathing frequency, the preset respiratory state information range may be in a range of 12 times/min-20 times/min, etc. Further, the preset respiratory state information range may also be a preset range corresponding to the breathing depth, the inhalation time, the exhalation time, the breathing smoothness, etc.

In some embodiments, the processor may determine the physical state of the human body based on at least one parameter of the HRV and the respiratory state information. In some embodiments, the respiratory state information may include the breathing frequency, and the processor may determine the physical state of the human body based on the HRV and the breathing frequency. In some embodiments, determining the physical state of the human body based on the HRV and the breathing frequency may include: in response to determining that the HRV of the physical state of the human body is not greater than a preset threshold and the breathing frequency is greater than an upper limit of a preset breathing frequency range, the physical state of the user being the tense state; in response to determining that the HRV of the physical state of the human body is not greater than the preset threshold and the breathing frequency is within the preset breathing frequency range, the physical state of the user being the relaxed state. In some embodiments, the respiratory state information may include the breathing depth, and the processor may determine the physical state of the human body based on the HRV and the breathing depth. In some embodiments, determining the physical state of the human body based on the HRV and the breathing depth may include: in response to determining that the HRV of the physical state of the human body is not greater than the preset threshold and the breathing frequency is less than a lower limit of the preset breathing depth range, the physical state of the user being the tense state; in response to determining that the HRV of the physical state of the human body is not greater than the preset threshold and the breathing depth is within the preset breathing depth range, the physical state of the user being the relaxed state. In some embodiments, the respiratory state information may include the inhalation time, and the processor may determine the physical state of the human body based on the HRV and the inhalation time. In some embodiments, determining the physical state of the human body based on the HRV and the inhalation time may include: in response to determining that the HRV of the physical state of the human body is not greater than the preset threshold and the inhalation time is less than a lower limit of a preset inhalation time range, the physical state of the user being the tense state; in response to determining that the HRV of the physical state of the human body is not greater than the preset threshold and the inhalation time is within the preset inhalation time range, the physical state of the user being the relaxed state. In some embodiments, the respiratory state information may include the exhalation time, and the processor may determine the physical state of the human body based on the HRV and the exhalation time. In some embodiments, the determining the physical state of the human body based on the HRV and the exhalation time may include: in response to determining that the HRV of the physical state of the human body is not greater than the preset threshold and the exhalation time is less than a lower limit of a preset exhalation time range, designating the physical state of the user as the tense state; and in response to determining that the HRV of the physical state of the human body is not greater than the preset threshold and the exhalation time is within the preset exhalation time range, designating the physical state of the user as the relaxed state. In some embodiments, the respiratory state information may include the breathing smoothness. The breathing smoothness may be calculated through the breathing frequency, the breathing depth, the inhalation time, and the exhalation time. The processor may determine the physical state of the human body based on the HRV and the breathing smoothness, etc. It should be noted that the processor may determine the physical state of the human body based on the HRV and a plurality of parameters of the respiratory state information, which are not repeated here.

In some embodiments, the processor may use at least the HRV and the respiratory state information as input data into a trained machine learning model, and the trained machine learning model may output the physical state of the human body based on the input data. Exemplary machine learning models may include a deep neural network model, a convolutional neural network model, etc. In some embodiments, the machine learning model may be obtained by training a large number of training samples with labels. Specifically, a plurality of training samples with labels may be input into an initial model, a loss function may be constructed based on an output of the initial model and the labels, and parameters of the model may be iteratively updated through training based on the loss function. In some embodiments, the training may be performed based on the plurality of training samples in various ways. For example, the training may be performed based on a gradient descent method. When a preset condition is met, the training may be ended and the trained model may be obtained. The preset condition may be that the loss function converges.

In some embodiments, the plurality of training samples may include a plurality of historical HRV data (e.g., *SDNN, SDANN, RMSSD, pNN*50, *SDNNi*, and other indicators) and a plurality of historical respiratory state information (e.g., any one or more of a historical breathing frequency, a historical breathing depth, a historical inhalation time, a historical exhalation time, a historical breathing smoothness, etc.). The indicator may be the physical state of the human body corresponding to each of the plurality of historical HRV data and each of the plurality of historical respiratory state information. The machine learning model can realize intelligent recognition of the physical state of the human body. In addition, the respiratory state information may be added to the model input, which increases the calculation circumference of the model, thereby improving the degree of fitting with an actual physiological condition. It is understood that parameters such as the HRV, the heart rate, the respiratory state information, etc. may be used as the input of the machine learning model, thereby further improving the degree of fitting with the actual physiological condition.

In some embodiments, different exercise movements may correspond to different HRVs. The processor may be further configured to evaluate the exercise movement based on the HRV and the exercise movement. The exercise movement may be determined based on the second strain sensor 930 in FIG. 9 and FIG. 10.

In some embodiments, the processor, a terminal device, or a database may pre-store a movement parameter and HRV data of at least one reference movement. After the processor determines the current exercise movement based on a lower limb movement parameter of the human body obtained by the second strain sensor 930, the processor may compare the movement parameter of the current exercise movement with the movement parameter of the reference movement, and compare the HRV of the current exercise movement with the HRV of the reference movement. In response to determining that a similarity between the HRV of the current exercise movement and the HRV of the reference movement is greater than a preset similarity threshold, an evaluation result is that the current exercise movement meets the standard of the reference movement (e.g., the current exercise movement is correct). In response to determining that the similarity between the HRV of the current exercise movement and the HRV of the reference movement is less than the preset similarity threshold, the evaluation result is that the current exercise movement does not meet the standard of the reference movement (e.g., the current exercise movement is incorrect or non-standard). For example, the processor may pre-store movement parameters such as a joint bending angle, a bending direction, etc. of yoga, Pilates, meditation, sitting meditation, standing, breathing training, etc., and HRVs corresponding to the above movements. In response to determining that a degree of fitting of a current joint bending angle and a current bending direction with the joint bending angle and the bending direction of the reference movement is not less than a first threshold (e.g., 90%), and a degree of fitting of the current HRV with the reference HRV is not less than a second threshold (e.g., 90%), the evaluation result is that the current movement meets the standard of the reference movement. In response to determining that the degree of fitting of the current joint bending angle and the current bending direction with the joint bending angle and the bending direction of the reference movement is less than the first threshold, and the degree of fitting of the current HRV with the reference HRV is less than the second threshold (e.g., 90%), the evaluation result is that the current movement does not meet the standard of the reference movement.

In some embodiments, the processor may send feedback information to the user through a feedback module. For example, in response to the evaluation result that the current exercise movement does not meet the standard of the reference movement, the processor may feed back a specific movement parameter to the user through the feedback module and provide a guidance plan, an exercise plan, etc.

In response to determining that the user is in the tense state, the user usually adjusts his or her HRV through relevant exercises (e.g., yoga, Pilates, meditation, sitting meditation, standing, and breathing training). In some embodiments, the processor may be further configured to determine whether the exercise movement achieves a preset effect based on the HRV and the exercise movement. The preset effect means that the HRV of the user is not less than a preset threshold. For example, in response to determining that the HRV of the user is not less than the preset threshold, the exercise movement may achieve the preset effect. In response to determining that the HRV of the user is less than the preset threshold, the exercise movement may not achieve the preset effect, and the processor may prompt the user to continue the current exercise movement or change the exercise movement through the feedback module until the HRV of the user is less than the preset threshold.

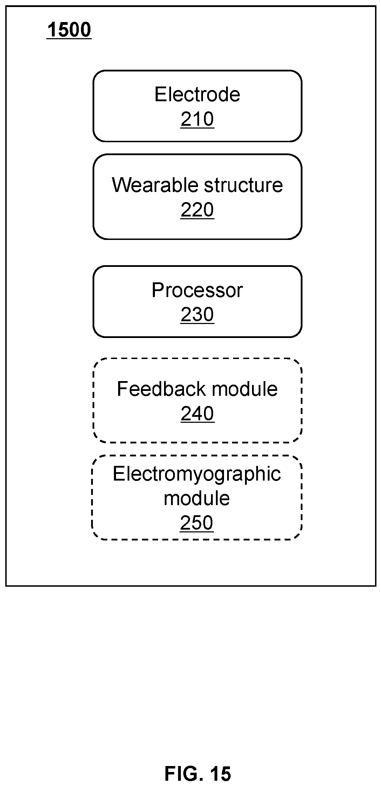

FIG. 15 is a schematic structural diagram illustrating another exemplary wearable device according to some embodiments of the present disclosure. As shown in FIG. 15, a wearable device 1500 may include at least two electrodes 210, a wearable structure 220, a processor 230, a feedback module 240, and an electromyographic module 250. The at least two electrodes 210, the wearable structure 220, and the processor 230 may be found in the related descriptions of FIG. 2.

In some embodiments, the wearable device may further include the feedback module 240. The feedback module may be in communication connection with the processor. The processor may be configured to issue a control instruction in response to a physical state of a human body. The feedback module may be configured to issue feedback information to a user based on the control instruction. The control instruction may be configured to determine whether the feedback module 240 sends the feedback information, and the specific content of the feedback information sent by the feedback module 240. Exemplary specific content of the feedback information may include a training plan, a movement prompt, a health reminder, etc.

In some embodiments, the feedback module 240 may be configured as a loudspeaker. The loudspeaker may be configured to control the loudspeaker to enter an operation state or switch an audio signal based on the control instruction. For example, in response to determining that the physical state of the human body is the tense state, the control instruction may be configured to determine that the feedback module 240 sends the feedback information and switch the audio signal to music with a soothing tune. In response to determining that the physical state of the human body is a relaxed state, the control instruction may be configured not to send the feedback information. The audio signal may be obtained via a network or may be pre-stored in a database.

In some embodiments, the feedback module 240 may be configured as other devices, such as an electronic screen, a vibration massage device, etc.

In some embodiments, after the loudspeaker enters a state or switches the audio signal, the processor may continue to determine the physical state of the human body based on at least an ECG signal. In response to determining that the physical state of the human body is still the tense state, the processor may switch the audio signal until the physical state of the human body enters the relaxed state. The processor may monitor the physical state of the human body in real time through an electrode, a first strain sensor, and a second strain sensor, and determine the feedback information based on a result of the real-time monitoring. For example, when the feedback module 240 plays a soothing music and the physical state of the human body is still the tense state, the processor may switch the music until the physical state of the human body enters the relaxed state.

In some embodiments, if the user is in the tense state when performing a target movement, the feedback module 240 may guide the user to switch the movement to adjust the user from the tense state to the relaxed state. After the physical state of the user is adjusted to the relaxed state, the feedback module 240 may remind the user to continue the target movement. Specifically, when the processor determines that the physical state of the user is the tense state, the processor may control the feedback module 240 to send the feedback information to the user. The feedback information may at least include information prompting the user to switch the movement. For example, when the user is doing yoga and the processor determines that the physical state of the user is the tense state, the processor may control the feedback module 240 to send a voice reminder to the user, reminding the user that the body is in the tense state, and recommend meditating or standing to adjust the physical state. In addition, the feedback module 240 may also guide the user to breathe to further improve the adjustment of the physical state of the user. When the body of the user is adjusted to the relaxed state, the user may be prompted to continue the yoga movement. In some embodiments, the feedback module 240 may guide the user to switch the movement in a manner of any one or more of a voice reminder, a text message, a video message, a vibration, an electric shock, etc.

In some embodiments, the physical state of the human body may be associated with the feedback information. When the physical state of the human body is the tense state, the feedback information may be information to relieve the tense state, such as a soothing music, a comfortable audio, a landscape image, guidance information (e.g., including information for guiding breathing adjustment, information for guiding movement adjustment, etc.), etc. In some embodiments, the movement of the human body may be associated with the feedback information. For example, when the movement of the human body is yoga, the feedback information may include guidance on the yoga movement, a training plan, a health reminder, and other information.

In some embodiments, the wearable device may further include the electromyographic module 250.

The electromyographic module 250 may be configured to collect an electromyographic signal of the human body. In some embodiments, the processor may determine whether the exercise movement of the human body is standard based on one or more of the electromyographic signal, the exercise movement and the breathing state information, the heart rate, or the HRV.

The electromyographic signal may be obtained from many parts of the human body, such as the calf, the thigh, the buttock, the waist, the back, the chest, the shoulder, the arm, the neck, etc. The electromyographic signal obtained from different parts may carry movement and function information of the corresponding parts. For example, an electromyographic signal from the leg may reflect the posture and movement state of the leg, such as walking, running, squatting, etc. Therefore, people's requirements for exercise and fitness guidance may be met by collecting the electromyographic signal through the wearable device. For example, when the human body is doing strength training, taking the dumbbell lateral raise as an example, and the arms of the human body are stretching out to two sides, the muscles (especially the shoulder muscles) exert force, the electromyographic signal is significantly enhanced, and the human body is generally in the inhalation state; when the arms are closed, the electromyographic signal is weakened, and the user is generally in the exhalation state. In this case, the various sub-movements in the lateral raise movement of the human body may be evaluated by determining the intensity of the electromyographic signal of the shoulder muscles of the human body under different breathing states, thereby determining whether each sub-movement is standard.

In some embodiments, the processor, a terminal device, or a database may pre-store one or more of the electromyographic signal, the exercise movement and the respiratory state information, the heart rate or the HRV of at least one reference movement. The processor may compare the above parameters of the current exercise movement with the above parameters of the reference movement. When a similarity between the parameters of the current exercise movement and the parameters of the reference movement is greater than a preset similarity threshold, an evaluation result is that the current exercise movement meets the standard of the reference movement, i.e., the exercise movement of the user is correct; when the similarity between at least one parameter of the current exercise movement and at least one parameter of the reference movement is less than the preset similarity threshold, the evaluation result is that the current exercise movement does not meet the standard of the reference movement, i.e., the current exercise movement of the user is incorrect or non-standard.

The beneficial effects of the embodiments of the present disclosure include but are not limited to the following content. (1) The HRV, the heart rate, the respiratory state information, and the exercise movement of the human body are monitored in real time through various components of the wearable device, thereby achieving real-time monitoring of the tense/relaxed state of the human body. (2) Monitoring components such as the electrodes and the strain sensors are integrated into the wearable device, and the impact of the monitoring components on the wearing comfort is reduced through the design of structure and fabric. (3) Parameters such as the HRV, the heart rate, and the respiratory state information are used as the model inputs to the machine learning model to increase the input circumference of the model and improve the degree of fitting between the model and the actual physiological condition. (4) Different content feedbacks are provided based on the monitoring result of the tense/relaxed state and the determination result of the exercise movement, thereby improving the user experience in different usage scenarios. (5) The wearable device can be applied to various application scenarios such as physical exercise, psychological testing, clinical health monitoring, movement correction, rehabilitation treatment, etc. It should be noted that different embodiments may produce different beneficial effects. In different embodiments, the beneficial effects that may be produced may be any one or a combination of the above, or any other beneficial effects that may be obtained.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment," or "one embodiment," or "an alternative embodiment" in various portions of this specification are not necessarily all referring to the same embodiment. Furthermore, the particular features, structures, or features may be combined as suitable in one or more embodiments of the present disclosure.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

**Claims**

1. A wearable device, comprising:

   at least two electrodes, configured to be attached to a human skin to collect an electrocardiograph (ECG) signal of a human body;
   a wearable structure, configured to carry the at least two electrodes and attach the at least two electrodes to two sides of a midsagittal plane of the human body; and
   a processor, configured to determine a heart rate variability (HRV) of the human body based on the ECG signal, and determine a physical state of the human body based at least on the HRV.

2. The wearable device of claim 1, wherein the physical state includes at least a relaxed state and a tense state, and the determining the HRV of the human body based on the ECG signal, and the determining the physical state of the human body based at least on the HRV includes:

   in response to determining that the HRV is not greater than a preset threshold, designating the physical state of the human body during exercise as the tense state; and

in response to determining that the HRV is greater than the preset threshold, designating the physical state of the human body during exercise as the relaxed state.

3. The wearable device of claim 1, wherein the determining the HRV of the human body based on the ECG signal, and the determining the physical state of the human body based at least on the HRV includes:
determining the HRV and a heart rate of the human body based on the ECG signal, and determining the physical state of the human body based on the HRV and the heart rate.

4. The wearable device of claim 3, wherein the physical state includes at least a relaxed state and a tense state, and the determining the physical state of the human body based on the HRV and the heart rate includes:

   in response to determining that the HRV is not greater than a preset threshold and the heart rate is greater than a preset heart rate threshold, designating the physical state of the human body during exercise as the tense state; and
   in response to determining that the HRV is greater than the preset threshold and the heart rate is not greater than the preset heart rate threshold, designating the physical state of the human body during exercise as the relaxed state.

5. The wearable device of claim 1, wherein the physical state includes at least a relaxed state and a tense state, and the determining the HRV of the human body based on the ECG signal, and the determining the physical state of the human body based at least on the HRV includes:
using at least the HRV as input data into a trained machine learning model, and the trained machine learning model outputting the physical state of the human body based on the input data.

6. The wearable device of claim 1, wherein the physical state includes at least a relaxed state and a tense state, and the determining the HRV of the human body based on the ECG signal, and the determining the physical state of the human body based at least on the HRV includes:

   obtaining a calibration curve, the calibration curve being obtained by fitting HRVs generated when the human body is in different physical states; and
   determining the physical state of the human body based on the calibration curve.

7. The wearable device of claim 1, further comprising a first strain sensor, wherein the first strain sensor is disposed on the wearable structure and fits with a waist region of the human body, and the first strain sensor is configured to collect respiratory state information of the human body based on a fluctuation of the waist region when the human body breathes.

8. The wearable device of claim 7, wherein the determining the HRV of the human body based on the ECG signal, and the determining the physical state of the human body based at least on the HRV includes:
determining the physical state of the human body based on the HRV and the respiratory state information.

9. The wearable device of claim 8, wherein the physical state includes at least a relaxed state and a tense state, and the determining the physical state of the human body based on the HRV and the respiratory state information includes:

   in response to determining that the HRV is greater than a preset threshold and the respiratory state information is within a preset range, designating the physical state of the human body during exercise as the relaxed state; and
   in response to determining that the HRV is not greater than the preset threshold and the respiratory state information is not within the preset range, designating the physical state of the human body during exercise as the tense state.

10. The wearable device of claim 8, wherein the physical state at least includes a relaxed state and a tense state, and the determining the physical state of the human body based on the HRV and the respiratory state information includes:
using at least the HRV and the respiratory state information as input data into a trained machine learning model, and the trained machine learning model outputting the physical state of the human body based on the input data.

11. The wearable device of any one of claims 1-10, further comprising a feedback module, wherein the feedback module is in communication connection with the processor, the processor is configured to issue a control instruction in response to the physical state of the human body, and the feedback module is configured to issue feedback information to a user based on the control instruction.

12. The wearable device of claim 11, wherein the feedback module includes a loudspeaker, and the loudspeaker is controlled to enter an operation state or switch audio signals based on the control instruction.

13. The wearable device of claim 12, wherein after the loudspeaker enters the operation state or switches the audio signals, the processor continues to determine the physical state of the human body at least based on the ECG signal, and in response to determining that the physical state of the human body is still the tense state, the processor switches the audio signals until the physical state of the human body enters into the relaxed state.

14. The wearable device of claim 11, wherein the physical state of the human body is associated with the feedback information.

15. The wearable device of claim 1, wherein the at least two electrodes are distributed on the wearable structure at interval, in response to determining that the human body wears the wearable structure, the at least two electrodes are located on two sides of a midsagittal plane of a waist region of the human body.

16. The wearable device of claim 15, wherein the at least two electrodes include a first electrode and a second electrode, wherein

    when the human body wears the wearable structure, the first electrode and the second electrode are respectively attached to an ilium, a rear waist, or an abdomen on the two sides of the midsagittal plane of the human body.

17. The wearable device of claim 16, wherein when the human body wears the wearable structure, the first electrode and the second electrode are symmetrically arranged with respect to the midsagittal plane of the human body.

18. The wearable device of claim 15, wherein the at least two electrodes include a first electrode, a second electrode, and a reference electrode, the first electrode and the second electrode are disposed on a surface of the wearable structure close to the human skin at interval, and the reference electrode is disposed between the first electrode and the second electrode.

19. The wearable device of claim 7, wherein the wearable structure is trousers, and the wearable device further comprises a second strain sensor, the second strain sensor is disposed at a position of the wearable structure corresponding to a leg or a buttock of the human body; and the processor is further configured to recognize an exercise movement of lower limbs of the human body based on the second strain sensor.

20. The wearable device of claim 19, wherein different exercise movements correspond to different HRVs, and the processor is further configured to evaluate the exercise movement based on the HRV and the exercise movement.

21. The wearable device of claim 19, comprising an electromyographic module, wherein the electromyographic module is configured to collect an electromyographic signal of the human body, and the processor is configured to evaluate the exercise movement of the human body based on the electromyographic signal, the exercise movement, and one or more of the respiratory state information, the heart rate, or the HRV.

<u>100</u>

120-1

130

110

120

Network

150

120-2

140

141    142    143

...

FIG. 1

**200**

Electrode
210

Wearable structure
220

Processor
230

FIG. 2

**300**

First extension direction

Second extension direction

320

311

312

FIG. 3

**FIG. 4**

**FIG. 5**

FIG. 6

701

7012          7011

7022          7021

702

（a）

701

7011          7012

7031          7032

703

（b）

FIG. 7

<u>800</u>

First extension direction

Second extension direction

820

811   813   812

**FIG. 8**

**FIG. 9**

900

910

923

921

922

930

FIG. 10

FIG. 11

**FIG. 12**

**FIG. 13**

HRV

Respiratory state information

Not greater than a preset threshold?

No

No

Within a preset respiratory state information range

Yes

Yes

Tense state

Relaxed state

**FIG. 14**

**1500**

Electrode
210

Wearable structure
220

Processor
230

Feedback module
240

Electromyographic
module
250

FIG. 15

## INTERNATIONAL SEARCH REPORT

| International application No. |
| --- |
| **PCT/CN2023/075666** |

**A. CLASSIFICATION OF SUBJECT MATTER**

A61B5/024(2006.01)i; A61B5/0245(2006.01)i; A61B5/0205(2006.01)i; A61B5/346(2021.01)i; A61B5/349(2021.01)i

According to International Patent Classification (IPC) or to both national classification and IPC

**B. FIELDS SEARCHED**

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT; ENTXTC; WPABSC; ENTXT; VEN; CNKI; 百度学术, BAIDU SCHOLAR: 心率变异, 放松, 紧张, 焦虑, 心电, 电极, 呼吸, 肌电, 阈值, HRV, ECG, EMG, relax, nervous, anxiety, stressful, electrode, respiration, threshold

**C. DOCUMENTS CONSIDERED TO BE RELEVANT**

| Category* | Citation of document, with indication, where appropriate, of the relevant passages | Relevant to claim No. |
| --- | --- | --- |
| X | CN 105496377 A (WU JIANKANG) 20 April 2016 (2016-04-20) description, paragraphs [0004]-[0060], and figures 1-4 | 1, 5-8, 10-21 |
| Y | CN 105496377 A (WU JIANKANG) 20 April 2016 (2016-04-20) description, paragraphs [0004]-[0060], and figures 1-4 | 2-4, 9, 11-14 |
| Y | CN 102488501 A (SHENZHEN CREATIVE INDUSTRY CO., LTD.) 13 June 2012 (2012-06-13) description, paragraphs [0002]-[0057], and figures 1-7 | 2, 9, 11-14 |
| Y | US 2020035337 A1 (FOLLOWFLOW HOLDING B. V.) 30 January 2020 (2020-01-30) description, paragraphs [0080]-[0081] | 3, 4, 11-14 |
| X | CN 204293140 U (WU JIANKANG) 29 April 2015 (2015-04-29) description, paragraphs [0002]-[0086], and figures 1-7b | 1, 5-8, 10-21 |
| A | CN 108209902 A (SHENZHEN FUTURE FITNESS CLOTHING TECHNOLOGY CO., LTD.) 29 June 2018 (2018-06-29) entire document | 1-21 |

☑ Further documents are listed in the continuation of Box C.  ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **16 June 2023** | **06 July 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/075666** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 111527494 A (B SAFETY LTD.) 11 August 2020 (2020-08-11)<br>entire document | 1-21 |
| A | CN 115137299 A (HUAWEI TECHNOLOGIES CO., LTD.) 04 October 2022 (2022-10-04)<br>entire document | 1-21 |

Form PCT/ISA/210 (second sheet) (July 2022)

**INTERNATIONAL SEARCH REPORT**
Information on patent family members

| | International application No. |
|---|---|
| | **PCT/CN2023/075666** |

| Patent document<br>cited in search report | | | Publication date<br>(day/month/year) | Patent family member(s) | | | Publication date<br>(day/month/year) |
|---|---|---|---|---|---|---|---|
| CN | 105496377 | A | 20 April 2016 | None | | | |
| CN | 102488501 | A | 13 June 2012 | CN | 102488501 | B | 24 April 2013 |
| US | 2020035337 | A1 | 30 January 2020 | None | | | |
| CN | 204293140 | U | 29 April 2015 | None | | | |
| CN | 108209902 | A | 29 June 2018 | None | | | |
| CN | 111527494 | A | 11 August 2020 | None | | | |
| CN | 115137299 | A | 04 October 2022 | None | | | |

Form PCT/ISA/210 (patent family annex) (July 2022)

**EP 4 509 043 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022132671 W